# EUROPEAN PATENT APPLICATION

(11) **EP 4 792 751 A2**
(43) Date of publication of application: **19.08.2026**
(21) Application number: 26173595.5
(22) Date of filing: 02.02.2024
(51) Int. Cl.: A61B 8/06

(54) **TRANSDUCER ASSEMBLIES TO OPTIMIZE BLOOD FLOW MONITORING**

(30) Priority: 03.02.2023 US 202363443301 P; 22.12.2023 US 202363614511 P
(62) Document number(s) of the earlier application(s) in accordance with Art. 76 EPC: 24155581.2 / 4 410 218
(71) Applicant: STRYKER CORPORATION, Portage, MI 49002-9711 (US)
(72) Inventor: SIEDENBURG, Clinton T., Kalamazoo, 49002 (US); PIRAINO, Daniel W., Kalamazoo, 49002 (US); TAYLOR, Tyson G., Kalamazoo, 49002 (US)
(74) Representative: Kilburn & Strode LLP

(57) **Abstract**

Ultrasound transducers optimized to detect blood flow are described. An example transducer includes a non-cubic piezoelectric crystal. An emitting side of the crystal is configured to emit ultrasound toward a blood vessel. A receiver is configured to detect a reflection of the ultrasound from blood in a blood vessel.

## Description

### CROSS-REFERENCE TO RELATED APPLICATION(S)

This application claims priority to U.S. Provisional App. No. 63/443,301 (titled "Blood Flow Monitors" and filed on February 3, 2023) as well as U.S. Provisional App. No. 63/614,511 (titled "Transducer Assemblies to Optimize Blood Flow Monitoring" and filed on December 22, 2023), each of which is incorporated by reference herein in its entirety.

### BACKGROUND

The velocity of blood flowing through a subject can be detected using Doppler ultrasound. An ultrasound beam can be transmitted through the subject's skin and toward blood flowing through the blood vessel. This beam can be referred to as an incident beam. When the ultrasound beam is reflected from cells in the moving blood, the reflection of the ultrasound beam has a frequency shift with respect to the incident beam. The frequency shift is dependent on the velocity of the moving blood. By detecting the frequency shift of the reflection of the incident beam, the velocity of the blood can be determined. Therefore, blood velocity can be detected using ultrasound.

### SUMMARY

According to an aspect is provided a transducer, comprising a non-cubic piezoelectric crystal comprising an emitting side configured to emit ultrasound toward a blood vessel, and a receiver configured to detect a reflection of the ultrasound from blood in a blood vessel.

Optionally, the non-cubic piezoelectric crystal comprises zirconate titanate, barium titanate, or lithium niobate.

Optionally, the non-cubic piezoelectric crystal has a polygonal cross-section. The polygonal cross-section can comprises greater than four sides. The emitting side of the non-cubic piezoelectric crystal can comprise one of the greater than four sides of the polygonal cross-section.

Optionally, the non-cubic piezoelectric crystal comprises a circular cross-section. The emitting side of the non-cubic piezoelectric crystal can comprise a curved side of the circular cross-section.

Optionally, the non-cubic piezoelectric crystal comprises a cross-section with a curved side. The emitting side of the non-cubic piezoelectric crystal can comprise the curved side of the cross-section.

Optionally, the non-cubic piezoelectric crystal is configured to emit the ultrasound in a first direction, and the blood in the blood vessel is flowing in a second direction that is non-perpendicular to the first direction.

Optionally, the transducer further comprises a housing configured to enclose the non-cubic piezoelectric crystal and the receiver. The housing can comprise a surface configured to contact skin. The first direction can be non-perpendicular to the surface of the housing. The non-cubic piezoelectric crystal can be configured to emit the ultrasound through the surface of the housing.

Optionally, the transducer further comprises an analog-to-digital converter (ADC) configured to generate a digital signal indicative of the reflection.

Optionally, the transducer further comprises a processor. The processor can be configured to determine a velocity of the blood by determining a difference between a frequency of the ultrasound emitted by the non-cubic piezoelectric crystal and a frequency of the reflection of the ultrasound from the blood in the blood vessel.

According to an aspect is provided a flow detector comprising a transducer as described above.

The flow detector can comprise a transducer comprising a piezoelectric crystal with a cross-section comprising a curved surface, the piezoelectric crystal being configured to emit ultrasound from the curved surface toward a blood vessel in a first direction. The transducer can comprise a receiver configured to detect a reflection of the ultrasound from blood flowing in a blood vessel in a second direction, the first direction being non-perpendicular to the second direction. The flow detector can comprise a processor configured to determine a velocity of the blood by analyzing the reflection. The flow detector can comprise a display configured to visually output an indication of the velocity of the blood.

Optionally, the flow detector further comprises a housing configured to enclose the piezoelectric crystal, the receiver, and the processor. The housing can comprise a surface configured to contact skin. The first direction can be non-perpendicular to the surface of the housing. The piezoelectric crystal can be configured to emit the ultrasound through the surface of the housing.

According to an aspect is provided a method comprising emitting, from a side of a non-cubic piezoelectric crystal, ultrasound toward a blood vessel and in a first direction. The method can comprise receiving a reflection of the ultrasound from blood flowing through the blood vessel in a second direction, the first direction being non-perpendicular to the second direction. The method can comprise determining a velocity of the blood by analyzing the reflection of the ultrasound from the blood flowing through the blood vessel. The method can comprise outputting an indication of the velocity.

Optionally, the non-cubic piezoelectric crystal has a polygonal cross-section, the polygonal cross-section comprising greater than four sides. The side of the non-cubic piezoelectric crystal can comprise one of the greater than four sides.

Optionally, the non-cubic piezoelectric crystal comprises a circular cross-section. The side of the non-cubic piezoelectric crystal can comprise a curved side of the circular cross-section.

Optionally, the non-cubic piezoelectric crystal comprises a cross-section with a curved side. The side of the non-cubic piezoelectric crystal can comprise the curved side of the cross-section.

Optionally, emitting, from the side of the non-cubic piezoelectric crystal, ultrasound toward the blood vessel and in the first direction comprises transmitting the ultrasound through a surface of a housing disposed on skin, the surface of the housing preferably being substantially parallel to the second direction.

Optionally, determining the velocity of the blood by analyzing the reflection of the ultrasound from the blood flowing through the blood vessel comprises determining a difference between a characteristic of the ultrasound and a characteristic of the reflection of the ultrasound, the characteristic e.g. being a frequency or rate of change of phase.

Optionally, outputting the indication of the velocity comprises determining that the velocity is above a first threshold or is below a second threshold, and optionally in response to determining that the velocity is above the first threshold or is below the second threshold, outputting an alert.

Optionally, outputting the indication of the velocity comprises outputting an audible signal indicating the velocity; outputting a visual signal indicating the velocity; and/or outputting, to an external device, a communication signal indicating the velocity.

It will be appreciated that any of the aspects, features and options described herein can be combined. It will particularly be appreciated that any of the aspects, features and options described in view of the transducer apply equally to the flow detector and method, and vice versa.

### BRIEF DESCRIPTION OF THE DRAWINGS

FIG. 1 illustrates an example environment for monitoring blood flow through one or more blood vessels of a subject.
FIG. 2 illustrates various placements of the flow monitor on the body of a subject.
FIG. 3 is a diagram illustrating a circuit for generating ultrasound that is non-perpendicular with blood flowing through a blood vessel.
FIGS. 4A and 4B illustrate examples of cylindrical arrays of transducer elements.
FIG. 5 illustrates an example of a transducer elements that collectively form a polygonal prism.
FIG. 6 illustrates an example of a planar transducer element configured to generate lobes of ultrasound that are transmitted in multiple directions.
FIG. 7 illustrates an example of a transducer element used with a wedge-shaped spacer.
FIG. 8 illustrates an example of a transducer element used with a layered spacer.
FIG. 9 illustrates an example of monitoring blood flow using needles.
FIG. 10 illustrates an example of a two-dimensional (2D) transducer element array that implements a phase delay (e.g., using analog or digital means) among the transducer elements.
FIG. 11 illustrates an example of a three-dimensional (3D) transducer element array.
FIG. 12 illustrates the structure of an example flow monitor.
FIG. 13 illustrates an example method for identifying a condition of a subject.
FIG. 14 illustrates an example method for providing feedback on a treatment performed on the subject.
FIG. 15 illustrates an example of an external defibrillator configured to perform various functions described herein.
FIG. 16 illustrates a chest compression device configured to perform various functions described herein.
FIG. 17 illustrates a flow monitor configured to perform various functions described herein.

### DETAILED DESCRIPTION

Previous Doppler ultrasound technologies have several deficiencies. First, ultrasound transducer assemblies generally emit ultrasound substantially perpendicularly to the surfaces configured to contact skin of the subject. However, this orients the ultrasound at a 90-degree angle with respect to blood flowing through vessels that are parallel to the skin. This physical arrangement prevents accurate blood flow measurements because it produces minimal Doppler shift of the reflected ultrasound.

Further, previous ultrasound devices are unsuited to emergency situations in which a subject is receiving chest compressions. During chest compressions, blood is pushed from the chest to the brain of the subject, even along veins. Thus, even if a user is monitoring the subject's blood flow using a previously known ultrasound transducer, the user may be unable to distinguish between blood flow from chest compressions and blood flow from heart-induced circulation.

Implementations of the present disclosure address these and other problems with previous technologies. Various implementations provide for a flow monitor configured to detect blood flow through one or more blood vessels of a subject. The flow monitor may be disposed on the skin of the subject and emit an incident beam (e.g., ultrasound and/or infrared light) at an angle that is non-parallel to the blood vessel(s) being monitored. In various implementations, the flow monitor detects the velocity of blood flowing through the blood vessel(s) by detecting a Doppler shift in a reflection of the incident beam from the blood. In some cases, the flow monitor detects the velocity of the blood without imaging the blood vessel(s).

In some implementations, the flow monitor detects blood flow through a paired artery and vein (e.g., an artery supplying blood to, and a vein receiving blood from, the same physiological structure), simultaneously. For instance, the flow monitor may detect blood flow through the carotid artery and jugular vein of the subject at the same time. By monitoring blood flow through both the artery and the vein, the flow monitor is configured to detect chest compressions administered to the subject, a return of spontaneous circulation (ROSC), pulseless electrical activity (PEA), net blood flow to a portion of the subject's body (e.g., the brain), or the like. In some cases, the flow monitor emits two incident beams that are respectively directed at the artery and vein. In some examples, the flow monitor emits a single incident beam that is reflected by the artery and the vein.

The flow monitor may provide feedback about a treatment being administered to the subject or a condition of the subject, based on the monitored blood flow through the blood vessel(s). For example, the flow monitor may direct a rescuer or mechanical chest compression device to change a position, frequency, duty cycle, or depth of chest compressions administered to the subject, or to pause or resume chest compressions, based on blood flow through the blood vessel(s). In some cases, the flow monitor detects an efficacy of chest compressions administered to the subject by detecting blood flow to the brain of the subject over time and/or by detecting blood flow within the brain of the subject. For instance, the flow monitor may detect blood flow by directing an incident beam through a temple, orbital cavity, or ear canal of the subject.

In some implementations, the flow monitor adjusts its analysis of the blood flow based on signals received from external devices. For instance, the flow monitor may temporally gate or filter blood flow measurements over time based on signals from a monitor-defibrillator, a mechanical chest compression device, an electrocardiogram (ECG), a blood oxygenation (e.g., regional oxygenation), or another type of sensor associated with the subject. The flow monitor, in some cases, is configured to remove a chest compression artifact from blood flow measurements based on communication from the mechanical chest compression device. In some cases, the flow monitor confirms a condition of the subject based on a combination of a blood flow measurement with another type of physiological parameter of the subject. As used herein, the term "physiological parameter," and its equivalents, may refer to an ECG, an impedance (e.g., a transthoracic impedance), a force administered to the subject, a blood pressure, an airway parameter (e.g., a partial pressure of carbon dioxide, a partial pressure of oxygen, a capnograph, an end tidal gas parameter, a flow rate, etc.), a blood oxygenation (e.g., a pulse oximetry value, a regional oximetry value, etc.), an electroencephalogram (EEG), a temperature, a heart sound, a blood flow rate, a physiological geometry (e.g., a shape of a blood vessel, an inner ear shape, etc.), a heart rate, a pulse rate, or another type of metric indicative of a condition of the subject.

In some implementations, the flow monitor includes other types of sensors configured to detect a condition of the subject. For instance, the flow monitor may include a microphone configured to detect a sound emitted by blood flowing through a blood vessel or the subject's heart pumping blood. In some cases, the flow monitor includes an accelerometer configured to detect a vibration or motion caused by blood flowing through a blood vessel of the subject.

As used herein, the terms "blood flow," "blood flow parameters," and their equivalents, may refer to one or more physiological parameters indicative of a movement of blood through a blood vessel. For example, the term "blood velocity" may refer a change in position with respect to time (i.e., distance per time, such as meters per second) of one or more blood cells moving in a blood vessel. The term "blood speed" may refer to a magnitude of a velocity (i.e., distance per time) of one or more blood cells moving in a blood vessel. The term "velocity profile," for example, may refer to a velocity of blood in a blood vessel with respect to a location within the blood vessel, such as a velocity of blood in a blood vessel with respect to a location along a cross-section of the blood vessel. The term "flow rate," and its equivalents, may refer to a volume or mass of blood that passes a boundary (e.g., a cross-section of a blood vessel) with respect to time. The terms "net flow," "net flow volume," and their equivalents, may refer to a volume or mass of blood that passes a boundary during a discrete time interval, such as during a cardiac cycle. A net flow volume can be calculated by integrating a flow rate over the time interval. Unless otherwise specified explicitly or by context, the term "blood flow" may refer to blood velocity, blood speed, velocity profile, flow rate, net flow, or any other flow-related parameter described herein.

Implementations of the present disclosure will now be described with reference to the accompanying figures.

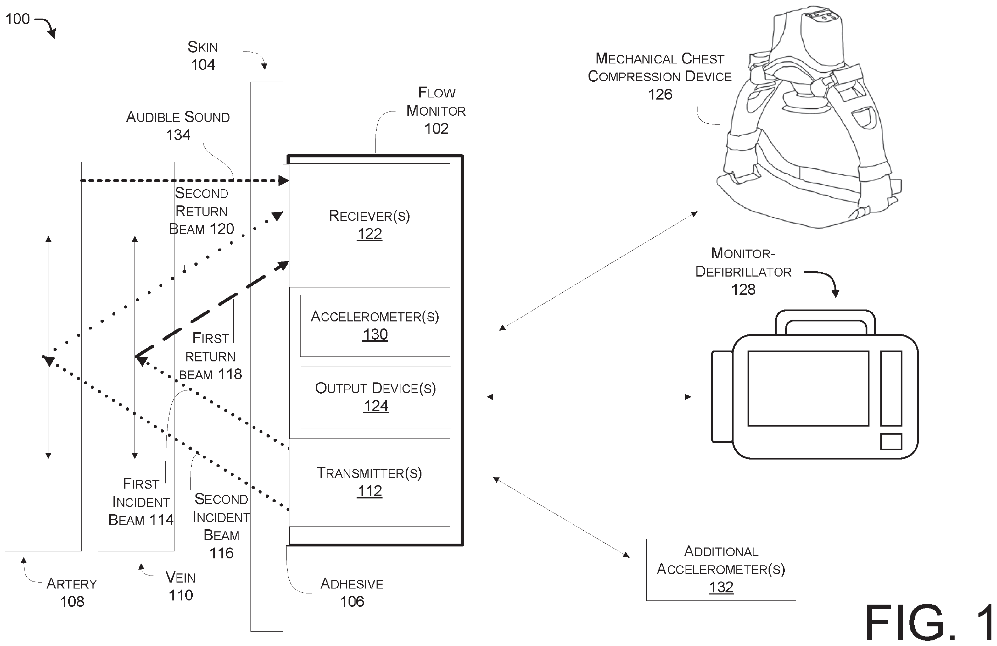

FIG. 1 illustrates an example environment 100 for monitoring blood flow through one or more blood vessels of a subject. A flow monitor 102 is adhered to skin 104 of the subject via an adhesive 106. The flow monitor 102, for instance, is located outside of the body of the subject. In some implementations, the flow monitor 102 is held on the skin 104 by a strap, a buckle, a bandage, or some other fastener. For instance, the flow monitor 102 may be wrapped around an extremity of the subject.

In various implementations, an artery 108 and a vein 110 are disposed underneath the skin 104. The artery 108 and the vein 110 are part of the circulatory system of the subject. The circulatory system includes a fluid circuit of various blood vessels (including the artery 108 and the vein 110). The subject includes a heart that, when functioning, pumps blood through the blood vessels. In particular, the heart moves blood from lungs of the subject, where the blood can be oxygenated, to other portions of the subject's body, such as the brain, other organs, and the subject's extremities. Along the circulatory system, cells within the blood deliver oxygen to cells of the subject, thereby supporting cellular respiration. In various cases, the artery 108 carries oxygenated blood from the lungs of the subject and the vein 110 carries deoxygenated blood toward the lungs. Examples of the artery 108 include a carotid artery, a subclavian artery, a coronary artery, a brachial artery, an iliac artery, a radial artery, a femoral artery, or a pulmonary artery. Examples of the vein 110 include a jugular vein, an iliac vein, a subclavian vein, a cephalic vein, a brachial vein, a basilic vein, a hepatic vein, a radial vein, an ulnar vein, a digital vein, a brachiocephalic vein, a femoral vein, a saphenous vein, a venous arch, or a tibial vein. In some cases, the pulmonary artery carries deoxygenated blood.

According to various implementations of the present disclosure, the flow monitor 102 is configured to detect the flow of blood through the artery 108 and/or vein 110. In particular cases, the flow monitor 102 includes one or more transmitters 112 configured to output one or more incident beams toward the artery 108 and/or vein 110. In the example illustrated in FIG. 1, the incident beams include a first incident beam 114 and a second incident beam 116.

In various cases, the first incident beam 114 and the second incident beam 116 include waves that are transmitted through the skin 104. The waves, for example, can be instantiated as light and/or sound. In various cases, the first incident beam 114 and the second incident beam 116 include at least one of infrared, near-infrared, or visible light. For instance, the light may have a frequency in a range of 300 GHz - 130 THz and a wavelength in a range of 700 nanometers (nm) to 1 millimeter (mm). For instance, the transmitter(s) 112 include one or more light sources, such as light-emitting diodes (LEDs) or lasers. In some examples, the transmitter(s) 112 may include one or more mirrors configured to split the light output by the light source(s), such as for an interferometric analysis. In some cases, the receiver(s) 122 include one or more light sensors, such as at least one of a photodiode, a phototransistor, a photomultiplier tube, a charge-coupled device, a metal-semiconductor-metal photodetector, or a complementary metal oxide semiconductor photodetector.

According to various implementations, the waves include ultrasound. As used herein, the term "ultrasound," and its equivalents, can refer to mechanical waves (e.g., in the form of pressure waves) having a frequency in a range of 20 kilohertz (kHz) to 200 megahertz (MHz). Ultrasound, for example, is sound in a frequency that is greater than an upper detection limit of a human ear. In various instances, transmitter(s) 112 include one or more piezoelectric crystals (including, e.g., lead zirconate titanate (PZT), LiNbO₃ (LN), lead magnesium niobate-lead titanate (PMN-PT), or lead indium niobate- lead magnesium niobate-lead titanate (PIN-PMN-PT)). When an electrical current is induced through the piezoelectric crystal(s), the piezoelectric crystal(s) vibrate at a frequency that produces ultrasound. In some examples, the transmitter(s) 112 include one or more micro-electromechanical system (MEMS) devices. In some instances, the transmitter(s) 112 include one or more capacitive micromachined ultrasonic transducers (CMUTs) and/or piezoelectric micromachined ultrasonic transducers (PMUT). Any electrical to mechanical conversion system or material that operates at the ultrasound frequency range would be suitable.

According to various implementations, the flow monitor 102 includes one or more ultrasound transducers. As used herein, the terms "ultrasound transducer," "ultrasonic transducer," "transducer," and their equivalents, may refer to a device that generates or detects ultrasound. For instance, the ultrasound transducer(s) include the transmitter(s) 112 and/or the receiver(s) 122. In some cases, an ultrasound transducer includes a transducer element (e.g., a piezoelectric crystal, MEMS device, or another type of electrical-to-mechanical conversion device), a first electrode disposed on one side of the transducer element, and a second electrode disposed on another side of the transducer element. In various implementations, the ultrasound transducer is configured to produce ultrasound by inducing a current through or a voltage between the first and second electrodes. In some cases, the ultrasound transducer is configured to detect ultrasound by detecting a current through or voltage between the first and second electrodes that is induced when the ultrasound is received by the transducer element. In some cases, the ultrasound transducer is encased in a housing, which may be watertight. The ultrasound transducer, in some examples, further includes a matching layer that is disposed between the transducer element and a surface of the housing from which an incident beam of ultrasound is emitted. The matching layer includes a material having an acoustic impedance that is between the acoustic impedance of the transducer element and an acoustic lens (if one exists or between the transducer and skin). In some implementations, a gel layer is disposed between the housing of the ultrasound transducer and the skin 104 that further matches the impedance between the ultrasound transducer and the body, thereby preventing the first incident beam 114 and the second incident beam 116 from being reflected by an interface containing air between the skin 104 and the ultrasound transducer. In some cases, the adhesive 106 serves as the gel layer.

In some examples, the first incident beam 114 is reflected and/or scattered by blood in the vein 110, thereby generating a first return beam 118. Further, in some cases, the second incident beam 116 is reflected and/or scattered by blood in the artery 108, thereby generating a second return beam 120. The flow monitor 102 includes one or more receivers 122 configured to detect the first return beam 118 and/or the second return beam 120.

As illustrated, the artery 108 and the vein 110 are substantially parallel to the skin 104. In various implementations of the present disclosure, the transmitter(s) 112 emit the first incident beam 114 and/or the second incident beam 116 in a direction that is non-perpendicular and non-parallel to the surface of the flow monitor 102 that is adhered to the skin 104. That is, the transmitter(s) 112 emit the first incident beam 114 and/or the second incident beam 116 in an angled fashion. Thus, a component of the first incident beam 114 is parallel to the blood flow through the vein 110 and/or a component of the second incident beam 116 is parallel to the blood flow through the artery 108.

Various implementations of the transmitter(s) 112 that enable angled transmission of the first incident beam 114 and the second incident beam 116 are disclosed herein. In some implementations in which the transmitter(s) 112 include one or more transducer elements configured to emit ultrasound, an individual transducer element may have the shape of a cylinder (with a height of the cylinder being substantially parallel to the skin 104 and perpendicular to a direction of the artery 108 and/or vein 110) or a polygonal prism (with a height of the polygonal prism being substantially parallel to the skin 104 and perpendicular to a direction of the artery 108 and/or vein 110). In some cases, a piezoelectric crystal may be divided into sections defined by an angle perpendicular to the height of the cylinder, each section serving as a different transducer element configured to emit ultrasound. In some cases, the transmitter(s) 112 include multiple (e.g., planar) transducer elements that emit respective ultrasound beams that are phase networked together such that they collectively form the angled first incident beam 114 or the second incident beam 116. In some cases, the transmitter(s) 112 include a single transducer element including portions that are insensitive to ultrasound (e.g., non-piezoelectric portions, such as including a polymer) and portions that are sensitive to ultrasound (e.g., piezoelectric portions), which can cause the first incident beam 114 or the second incident beam 116 to be output as a grating lobe. The grating lobe may include components emitted at different angles, at least one of which may include the first incident beam 114 or the second incident beam 116 to be transmitted at an angle with respect to the artery 108 or vein 110.

In some implementations, the transmitter(s) 112 include a transducer element that emits ultrasound from a surface that is nonparallel to the skin 104. For example, the flow monitor 102, in some cases, includes a wedge-shaped spacer between the crystal and the skin 104. The spacer may be substantially acoustically transparent. In some examples, the wedge-shaped spacer includes a polymer or gel that is configured to perform impedance matching between the crystal and the skin 104. For instance, the spacer may include multiple layers arranged in steps that are configured to be in contact with the crystal and/or tilt the crystal with respect to the skin 104.

In some cases, the flow monitor 102 includes an acoustic "lens" that is disposed between a crystal emitting ultrasound and the skin 104. For example, the acoustic lens can be a spacer with a nonuniform acoustic impedance. Thus, the acoustic lens may bend the ultrasound emitted by the crystal before it is transmitted through the skin 104.

In some examples, the transmitter(s) 112 includes a crystal that is disposed inside of a needle that is disposed through the skin 104. In some implementations, the transmitter(s) 112 include an array of crystals configured to emit the first incident beam 114 and/or the second incident beam 116 through the skin 104. For example, the array may be arranged on a curved surface, such that individual crystals may point in different directions. In operation, the flow monitor 102 may automatically determine which incident beams whose return beams produce a greatest frequency and/or phase shift with respect to the incident beams, and may define those beams as the first incident beam 114 and/or the second incident beam 116.

In various implementations, the first return beam 118 may represent a frequency and/or phase shift with respect to the first incident beam 114 due to the Doppler effect. Similarly, because the component of the second incident beam 116 is parallel to the blood flow through the artery 108, the second return beam 120 may represent a frequency and/or phase shift with respect to the second incident beam 116 due to the Doppler effect. These shifts occur due to the Doppler effect and may be referred to as "Doppler shifts."

According to various implementations, the flow monitor 102 determines a velocity of the blood flow through the artery 108 and the vein 110 due to a difference between the frequencies of the first incident beam 114 and the first return beam 118, as well as a difference between the frequencies of the second incident beam 116 and the second return beam 120. The transmitter(s) 112, in some cases, operate in a continuous wave Doppler mode (also referred to as "CW Doppler"), and continuously transmit the first incident beam 114 and the second incident beam 116 during a monitoring period. The receiver(s) 122, for instance, continuously detect the first return beam 118 and the second return beam 120 during the monitoring period. For instance, the flow monitor 102 detects the blood velocity in the artery 108 and the blood velocity in the vein 110, in-real time, continuously or semi-continuously based on the frequency shifts between the first incident beam 114 and the first return beam 118 as well as between the second incident beam 116 and the second return beam 120.

In various implementations, the flow monitor 102 operates in a pulsed-wave Doppler mode (also referred to as "PW Doppler"). For instance, the flow monitor 102 causes the incident beam 116 to output the first incident beam 114 and/or the second incident beam 116 in pulses and detects the first return beam 118 and/or the second return beam 120 as return pulses. In various cases, a time delay between the output pulses and the return pulses is indicative of the depth of a structure from which the return pulses are reflected. In various implementations, the flow monitor 102 can determine the blood velocity in the artery 108 based on phase shifts between the pulses of the first incident beam 114 and the first return beam 118. Further, the flow monitor 102 can determine the blood velocity in the vein 110 based on phase shifts between the pulses of the second incident beam 116 and the second return beam 120.

In some cases, the flow monitor 102 detects the blood velocities at a sampling rate that corresponds to at least twice the component of the velocity range in the direction of the beam pointing angle of the first incident beam 114 and/or the second incident beam 116.

In some examples, the flow monitor 102 performs laser Doppler velocimetry in order to detect the blood velocity. In various cases, the flow monitor 102 includes one or more interferometric sensors. For example, the first incident beam 114 and the second incident beam 116 are light beams (e.g., coherent light beams) that are split (e.g., by one or more mirrors) prior to transmission through the skin 104. The flow monitor 102 may detect the blood velocities in the artery 108 and the vein 110 by comparing the first return beam 118 and the second return beam 120 to the split beams generated from the first incident beam 114 and the second incident beam 116. Techniques for interferometric detection of blood velocity can be found in, for example, R. D. Rader, C. M. Stevens and J. P. Meehan, "An Interferometric Blood Flow Measurement Technique - A Brief Analysis," in IEEE Transactions on Biomedical Engineering, vol. BME-21, no. 4, pp. 293-297, July 1974; Nagahara, et al., Method. Invest. Ophthalmol. Vis. Sci. 2011;52(1):87-92; and Robinson, et al., Sci Rep 13, 8803 (2023), each of which is incorporated by reference herein in its entirety.

In some cases, the flow monitor 102 images a portion of the subject that includes the artery 108 and the vein 110. In some cases, the flow monitor 102 generates an image using the first return beam 118 and the second return beam 120 using one or more sonographic techniques. In various implementations, the flow monitor 102 generates the image using multiple return beams including the first return beam 118 and the second return beam 120. The flow monitor 102, in some implementations, generates the multiple return beams by sweeping the first incident beam 114 and the second incident beam 116 across a section of the subject being imaged. For example, the flow monitor 102 may generate a real-time image of the subject that includes cross-sections of the artery 108 and vein 110, respectively. In some implementations, the flow monitor 102 automatically segments the cross-sections of the artery 108 and vein 110 in the real-time image. In some cases, the flow monitor 102 performs segmentation of a scrolling Doppler image (e.g., Doppler shift in a y-axis is swept in time along an x-axis) to segregate out the Doppler information of the vein 110 from the artery 108. Various types of segmentation techniques can be used, such as detection using histogram of oriented gradients (HOG) features, a scale-invariant feature transform (SIFT), Viola-Jones object detection framework, or You Only Look Once (YOLO). Once the cross-sections depicted in the image are identified using image segmentation, the flow monitor 102 can further classify the cross-sections using a support vector machine (SVM). In some cases, the flow monitor 102 uses one or more trained convolutional neural networks (CNNs) to segment and/or classify the cross-sections of the artery 108 and the vein 110 depicted in the image. In various cases, the flow monitor 102 may differentiate the cross-section corresponding to the artery 108 an the cross-section corresponding to the vein 110.

In particular implementations, the flow monitor 102 detects the velocity of the blood through the artery 108 and the velocity of the blood through the vein 110, simultaneously. For example, the first return beam 118 may be reflected from the artery 108 and the second return beam 120 may be reflected from the vein 110, so that the flow monitor 102 can detect the blood velocity of the artery 108 based on the first return beam 118 and may detect the blood velocity of the vein 110 based on the second return beam 120.

Simultaneously monitoring the blood velocity through the artery 108 and the vein 110 may enable certain evaluations of the subject. In some implementations, the flow monitor 102 detects a volumetric flow rate or net flow volume (e.g., during a time period) through at least a portion of the subject based on the blood velocity through the artery 108 and the vein 110. In some cases, the flow monitor 102 determines the volumetric flow rate through the artery 108 by integrating the velocity of the blood through the artery 108 across a cross-sectional area of the artery 108. The flow monitor 102 may determine the volumetric flow rate through the vein 110 by integrating the velocity of the blood through the vein 110 across a cross-sectional area of the vein 110. In various implementations, the flow monitor 102 is configured to detect a net flow volume that flows through the cross-section of the artery 108 during a time period (e.g., a cardiac cycle, a chest compression cycle, a portion of a chest compression cycle, or any combination thereof) by integrating the volumetric flow rate through the artery 108 over the time period. Similarly, the flow monitor 102 is configured to detect a net flow volume that flows through the cross-section of the vein 110 during the time period by integrating the volumetric flow rate through the vein 110 over the time period.

The artery 108, for instance, supplies oxygenated blood to the portion of the subject, and the vein 110 transports deoxygenated blood from the portion of the subject. In some examples, the flow monitor 102 is configured to detect a net flow volume of blood to a portion of the subject's body over time. In particular cases, the artery 108 is a carotid artery and the vein 110 is a jugular vein, and the flow monitor 102 is configured to detect a net flow volume of blood to the brain of the subject over time.

The flow monitor 102 may assess a condition of the subject and/or evaluate a treatment administered to the subject by analyzing blood flow parameters (e.g., velocity, volumetric flow rate, or net flow volume of blood) in the artery 108 and/or the vein 110. In some implementations, the flow monitor 102 identifies chest compressions performed on the subject based on the blood velocity through the artery 108 and/or the blood velocity through the vein 110. When (e.g., effective) chest compressions are performed on the subject, the compressions push blood through the artery 108 and the vein 110 in a direction that moves distally from the chest. Thus, chest compressions cause blood flowing through the artery 108 and blood flowing through the vein 110 to travel in parallel directions. Moreover, an individual chest compression causes the blood to flow in the artery 108 and vein 110 in a surge. Thus, in some cases, the flow monitor 102 detects that chest compressions are being performed on the subject by detecting that the blood through the artery 108 and the blood through the vein 110 is traveling in the same or parallel directions (e.g., in a direction pointing distally from the chest). For instance, the flow monitor 102 can detect whether chest compressions are undesirably moving blood in the same direction through the artery 108 and the vein 110.

In some cases, the flow monitor 102 provides feedback about the efficacy of the chest compressions based on the blood velocity through the artery 108 and the vein 110 and/or the net flow volume to the portion of the subject. For instance, if the flow monitor 102 detects less than a threshold blood velocity through the artery 108 or vein 110, or detects less than a threshold net flow volume to the portion of the subject (e.g., during a particular time period), the flow monitor 102 can output a feedback signal via one or more output devices 124. The output device(s) 124, for instance, include a display (e.g., a screen configured to visually output signals), a speaker (e.g., configured to audibly output signals), a haptic feedback device (e.g., configured to convey signals by vibrating or otherwise moving), a transceiver (e.g., configured to transmit signals to external devices), or any combination thereof. In some cases, the feedback signal is output to a rescuer (not illustrated) performing the chest compressions, and may cause the rescuer to adjust the depth of the chest compressions, adjust the frequency of the chest compressions, or adjust the position of the chest compressions relative to the subject's chest.

In some implementations, the feedback signal is transmitted to a mechanical chest compression device 126 that is performing the chest compressions. The mechanical chest compression device 126, in some cases, administers the chest compressions by moving a plunger up and down on the subject's chest. The feedback signal, for instance, causes the mechanical chest compression device 126 to adjust the depth of the chest compressions, adjust the frequency of the chest compressions, adjust the position of the plunger relative to the subject's chest, pause chest compressions, or initiate chest compressions. In some implementations, the mechanical chest compression device 126 transmits a signal to the flow monitor 102 that indicates the timing, frequency, position, or another chest compression parameter characterizing the chest compressions administered by the mechanical chest compression device 126. The flow monitor 102, in some cases, generates the feedback signal based on the chest compression parameter, such that the feedback signal is specific to the conditions reported by the mechanical chest compression device 126.

The placement of chest compressions, in particular, impacts the flow of blood through specific blood vessels through the subject's body. In some cases, the flow monitor 102 enables optimization of the position of the chest compressions based on the flow rate through the artery 108, the flow rate through the vein 110, the net flow volume through the portion of the subject's body, a current position of the chest compressions, an identity or position of the artery 108 in the subject's body, an identity or position of the vein 110 in the subject's body, or any combination thereof. For example, if the chest compressions are being administered to the right of an ideal position, the flow monitor 102 may detect a peak and/or mean flow rate of blood in the artery 108 that is below a threshold. In various implementations, the flow monitor 102 (or another computing device that is communicatively coupled to the flow monitor 102) generates a feedback signal including an instruction to apply the chest compressions one direction or another and/or closer to the ideal position.

According to various implementations, the flow monitor 102 can detect the presence of spontaneous circulation of the subject, in which the heart of the subject is spontaneously pumping a sufficient amount of blood through the body of the subject. In some cases, the flow monitor 102 detects a return of spontaneous circulation (ROSC). The flow monitor 102, for instance, can detect spontaneous circulation while the subject is receiving chest compressions. As noted previously, when the subject is receiving chest compressions, the chest compressions cause parallel or unidirectional blood flow in the artery 108 and the vein 110. In contrast, the pumping heart of the subject circulating blood through the subject's circulatory system causes antiparallel or bidirectional blood flow in the artery 108 and the vein 110. Thus, the flow monitor 102, in some cases, detects spontaneous circulation in response to detecting antiparallel or bidirectional blood flow in the artery 108 and vein 110.

In some cases, the flow monitor 102 detects spontaneous circulation using other techniques. In general, the heart is capable of pumping blood at a greater peak velocity than chest compressions. Therefore, the monitor 102 may detect spontaneous circulation by detecting that a blood velocity through the artery 108 or the vein 110 is greater than a threshold. Further, the blood velocity through the artery 108 or the vein 110 with respect to time during chest compressions is different than the blood velocity through the artery 108 or the vein 110 with respect to time during spontaneous circulation. According to some implementations, the flow monitor detects spontaneous circulation by determining that at least one frequency component of the volumetric flow rate over time through the artery 108 or vein 110 has changed. For instance, a shape or morphology of the volumetric flow rate over time can be indicative of spontaneous circulation.

Certain functionalities of the flow monitor 102 are enhanced by communications with other devices, such as the mechanical chest compression device 126 and a monitor-defibrillator 128. Collectively, any combination of the flow monitor 102, the mechanical chest compression device 126, and the monitor-defibrillator 128 may be a resuscitation system that is configured to resuscitate a single subject. For example, the flow monitor 102 is configured to modify and/or temporally gate measurements it performs based on communications from the mechanical chest compression device 126 and/or the monitor-defibrillator 128. In some cases, the flow monitor 102 receives a communication signal from the mechanical chest compression device 126 that indicates a frequency of chest compressions administered by the mechanical chest compression device 126, a timing of chest compressions administered by the mechanical chest compression device 126, or a time of a pause in the chest compressions administered by the mechanical chest compression device 126. In some cases, the flow monitor 102 is configured to remove a chest compression artifact from a blood velocity over time based on the communication signal from the mechanical chest compression device 126. For instance, the flow monitor 102 may remove an artifact from the blood velocity by applying a band reject filter centered around the chest compression frequency or by applying a comb filter that includes the chest compression frequency and one or more harmonics of the chest compression frequency. In some cases, the flow monitor 102 is configured to temporally gate a blood velocity measurement during a time window in which the mechanical chest compression device 126 has paused chest compressions or between chest compressions performed by the chest compression device 126. Using these techniques, in some cases, may enable the flow monitor 102 to more accurately identify spontaneous circulation of the subject by distinguishing between blood flow caused by the chest compressions and spontaneous blood flow induced by the heart of the subject.

In some cases, the monitor-defibrillator 128 transmits a communication signal to the flow monitor 102 indicating a time at which the monitor-defibrillator 128 is administering a treatment to the subject, such as an electrical shock or pace pulses. Because the electrical shock or pace pulses can interfere with the accuracy of other measurements performed by the flow monitor 102, the flow monitor 102 may gate the measurements at a time interval that omits the treatment to the subject. In some implementations, the flow monitor 102 includes sensitive electronics that could potentially be damaged when the treatment is administered to the subject. According to some examples, the flow monitor 102 includes a circuit with at least one switch that disconnects or otherwise shields the sensitive electronics during the treatment.

In some examples, the flow monitor 102 includes or is otherwise communicatively coupled to devices that include one or more sensors (not illustrated) configured to detect other physiological parameters. For example, the flow monitor 102 includes or is communicatively coupled with a sensor configured to detect an electrocardiogram (ECG), an oximetry sensor (e.g., a regional oximetry sensor, a pulse oximetry sensor, a cerebral oximetry sensor, or the like), or both.

In various implementations, the flow monitor 102 includes one or more accelerometers 130 configured to detect an acceleration of the flow monitor 102 and/or the skin 104 of the subject. The acceleration, for instance, is indicative of chest compressions administered to the subject or a pulse of the subject through the artery 108 or vein 110. Thus, in some cases, the flow monitor 102 detects whether the subject has a pulse based on the acceleration detected by the accelerometer(s) 130. At least one additional accelerometer 132 may further be disposed on another portion of the subject (e.g., the subject's chest) and may be configured to detect an acceleration indicative of the chest compressions. The flow monitor 102, in some implementations, removes a chest compression artifact of the acceleration detected by the accelerometer(s) 130 over time based on the acceleration detected by the additional accelerometer(s) 132 over time. In various implementations, the flow monitor 102 is configured to accurately detect spontaneous circulation or another condition of the subject based on the detected accelerations.

As used herein, the term "measurement" may refer to a blood velocity, a net blood flow volume, an ECG, an oxygenation of the subject's blood, an acceleration, or another physiological parameter of the subject. In some cases, the flow monitor 102 validates and/or temporally gates a first measurement in view of a second measurement. For example, if the ECG indicates that the heart of the subject is beating, but the blood velocity or net blood flow indicates that oxygenated blood is not circulating in the subject, then the flow monitor 102 may refrain from indicating that the subject has spontaneous circulation. In some cases, the flow monitor 102 may output a signal indicating that the subject has pulseless electrical activity (PEA).

In various cases, the flow monitor 102 is configured to detect blood flow in the brain of the subject. In some cases, the artery 108 is a part of the circle of Willis of the subject. If the subject is an adult, the skull of the subject may highly attenuate the first incident beam 114, the second incident beam 116, the first return beam 118, and the second return beam 120. For instance, the skull may have high attenuation with respect to ultrasound and/or light (e.g., at certain frequencies). Thus, in various examples, the flow monitor 102 is configured to direct the first incident beam 114, the second incident beam 116, the first return beam 118, and the second return beam 120 through a window in the skull. For instance, the flow monitor 102 is configured to monitor the artery 108 and/or vein 110 through a temple of the subject, an orbital socket of the subject, or an ear canal of the subject. In some cases, the flow monitor 102 is strapped to the head of the subject, such as in the form of an eyepatch.

In some cases, the operation of the flow monitor 102 is optimized in other ways to enable monitoring within the brain. For instance, if the first incident beam 114 and the second incident beam 116 include ultrasound, the ultrasound may have a frequency of less than 1 MHz. Although sub MHz ultrasound is incapable of generating high-resolution images, in some cases, the flow monitor 102 detects the flow through the artery 108 or vein 110 without generating an image of the artery 108 or vein 110. That is, sub MHz ultrasound is sufficient for Doppler flow detection in various implementations described herein.

Another way in which the flow monitor 102 is optimized for monitoring with the brain relates to the type of the artery 108 and/or vein 110 monitored by the flow monitor 102. Attenuation of an incident beam increases as it travels through an attenuating structure. Thus, in some cases, a distance between the flow monitor 102 (e.g., disposed on the skin 104 or eye) is less than a threshold distance.

In particular cases, it may be difficult to differentiate specific blood vessels in the brain (e.g., within the Circle of Willis) in which the first return beam 118 and/or the second return beam 120 are reflected and/or scattered. Further, it may be difficult to identify angles from which the blood vessels in the brain are disposed with respect to the flow monitor 102. Accordingly, it may be difficult to accurately quantify blood flow parameters of a blood vessel in the brain using techniques described herein. However, even detecting the presence or absence of movement of blood in the brain can provide helpful feedback in order to detect chest compression efficacy and/or spontaneous circulation. According to various implementations, the flow monitor 102 outputs an indication if the flow monitor 102 detects movement, or the absence of movement, or the relative difference of movement between the compression and decompression parts of CPR or the diastolic and systolic parts of a normal sinus rhythm of blood in the brain of the subject.

By detecting blood flow in the brain, the flow monitor 102 is configured to detect an efficacy of chest compressions being administered to the subject. Technologies that monitor blood flow in extremities can indirectly detect chest compression efficacy. However, because a primary purpose of chest compressions is to provide oxygenated blood to the brain of the subject, the flow monitor 102 is able to directly detect the efficacy of the chest compressions by monitoring blood flow in the brain. The flow monitor 102, for instance, can output a feedback signal (e.g., to the mechanical chest compression device 126 or to the user) indicating whether chest compressions are generating blood flow in the brain. In some cases, the feedback signal includes a metric indicative of the blood flow in the brain (e.g., a quantity that increases with an increase in a detected blood velocity or flow rate).

According to some examples, the receiver(s) 122 include one or more microphones configured to detect an audible sound 134 from the subject. For example, the flow monitor 102 may include the functionality of a sophisticated stethoscope. In some cases, the microphone(s) detect the audible sound 134 emitted by blood flowing through the artery 108 or vein 110. In some examples, the microphone(s) detect the audible sound 134 emitted by the heart of the subject. In various implementations, the audible sound 134 can be in an audible or inaudible range. The flow monitor 102, in various cases, detects the audible sound 134 at a sampling frequency, such as a sampling frequency of greater than 100 Hz, 1 kHz, or 10 kHz. In some implementations, the flow monitor 102 determines a condition of the subject based on the audible sound 134.

According to some cases, the output device(s) 124 include a speaker that outputs an audible signal indicative of the audible sound 134 detected by the flow monitor 102. For example, the output device(s) 124 output the sampled audible sound 134 into an audio headset of the user, or in an environment in which the user is present, at a volume that can be perceived by the user in an emergency scene. That is, the output device(s) 124 may amplify the audible sound 134. Some monitors output computer-generated sounds indicative of a heart rate of a subject, such as a "beep" sound whenever a QRS complex is detected in an ECG. According to some implementations, the audible sound 134 includes more diagnostic-relevant information that can be perceived by the user than a computerized "beep" sound. In some examples, a condition of the subject can be identified using the strength, velocity, or morphological characteristics of blood flow, but these features cannot necessarily be identified using audio output from a previous type of monitor. Furthermore, by outputting an amplified version of the audible sound 134 as an audible signal, the flow monitor 102 may convey potentially important information about the condition of the subject without visually outputting it on a display. The user, for example, may be monitoring other visual signals on the monitor-defibrillator 128, and thus the flow monitor 102 may enable cognitive offloading for the user.

In some implementations, the receiver(s) 122 include multiple microphones configured to detect audible sounds 134 from multiple locations on the subject's body. For instance, the microphones may detect the audible sound 134 from a right quadrant of the subject's chest and another audible sound 134 from a left quadrant of the subject's chest. The flow monitor 102, in some cases, analyzes the detected sounds for features indicative of a medical condition. For example, the flow monitor 102 detects the medical condition by detecting an anomaly in the detected sound. In some implementations, the flow monitor 102 selectively outputs a single one of the audible sounds based on an input signal received from the user. In some cases, the flow monitor 102 selectively outputs a single one of the audible sounds that is indicative of the medical condition. In various implementations, the output device(s) 124 are configured to output a signal indicating the medical condition.

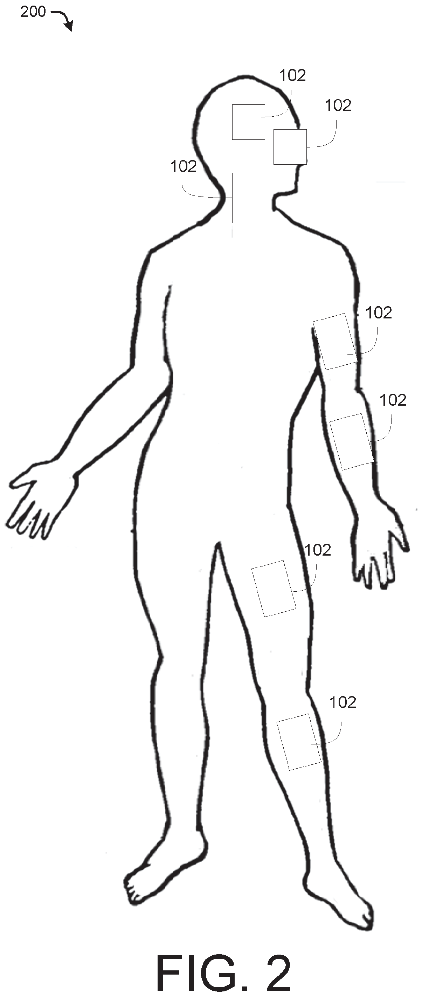

FIG. 2 illustrates various placements of the flow monitor 102 on the body of a subject 200. For example, the flow monitor 102 may be disposed on a temple, an orbital socket, an ear canal, a neck, an upper arm, a lower arm, an upper leg, or a lower leg of the subject 200. In some implementations, the flow monitor 102 includes multiple physical devices that are disposed on different parts of the body of the subject 200, simultaneously. In various implementations, the flow monitor 102 may be integrated into a cot or other support on which the subject 200 is disposed.

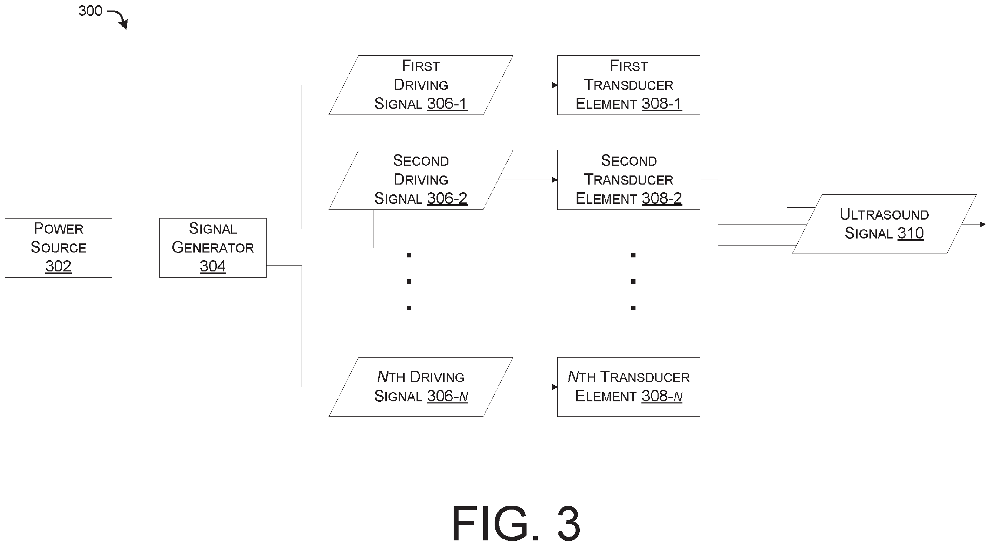

FIG. 3 is a diagram illustrating a circuit 300 for generating ultrasound that is non-perpendicular with blood flowing through a blood vessel. In some cases, the circuit 300 is incorporated into a flow monitor, such as the flow monitor 102 described above with reference to FIGS. 1 and 2.

The circuit 300 includes a power source 302 configured to output power. In some implementations, the power source 302 includes a battery, capacitor, or other power storage device. In some implementations, the power source 302 is configured to output a voltage and/or current.

The circuit 300 further includes a signal generator 304 configured to generate analog signals with power from the power source 302. That is, the signal generator 304 is configured to output first to *n*th driving signals 306-1 to 306-*n*, wherein *n* is a positive integer. The signal generator 304, in various cases, includes analog and/or digital circuitry. For example, the signal generator 304 includes one or more drive circuits. In some cases, the signal generator 304 includes one or more power amplifiers. Examples of power amplifiers include linear power amplifiers (e.g., Class A, B, AB, or C), switching power amplifiers (e.g., any of Classes D through T). Each of the first to *n*th driving signals 306-1 to 306-*n* may have a respective phase, amplitude, and waveform.

First to *n*th transducer elements 308-1 to 308-*n* are respectively configured to receive the first to *n*th driving signals 306-1 to 306-*n* from the signal generator 304. In some examples, each of the first to *n*th transducer elements 308-1 to 308-*n* includes one or more piezoelectric elements electrically connected to electrodes that deliver the first to *n*th driving signals 306-1 to 306-*n* to the piezoelectric element(s), for instance. According to various examples, each piezoelectric element includes a crystal and/or ceramic. In some cases, at least one piezoelectric micromachined ultrasonic transducer (PMUT) element is included in the first to *n*th transducer elements 308-1 to 308-*n*. According to some cases, the first to *n*th transducer elements 308-1 to 308-*n* include one or more capacitive micromachined ultrasonic transducer (CMUT) elements.

Based on the first to nth driving signals 306-1 to 306*-n*, the first to nth transducer elements 308-1 to 308*-n* collectively output an ultrasound signal 310. In various cases, the ultrasound signal 310 is transmitted at an angle with respect to a housing of a device including the first to nth transducer elements 308-1 to 308*-n* and/or skin of a subject into which the ultrasound signal 310 is transmitted. That is, the ultrasound signal 310 may be non-perpendicular and nonparallel to the housing and/or the skin. Accordingly, when the device is applied to the skin of a subject, the ultrasound signal 310 may be non-perpendicular to the flow of blood through a blood vessel located below the skin.

According to some examples, an example driving signal among the first to *n*th driving signals 306-1 to 306-*n* varies periodically from ground, to a positive voltage, to a negative voltage, to ground. For example, the example driving signal can be achieved by a field effect transistor (FET), such as a metal-oxide-semiconductor FET (MOSFET), included in the signal generator 304. A frequency of the periodic signal, for instance, is within a threshold frequency of a resonant frequency of an example transducer element (among the first to nth transducer elements 308-1 to 308-*n*) that receives the example driving signal. In some examples, each one of the first to *n*th driving signals 306-1 to 306-*n* has similar characteristics to the example driving signal.

In some implementations, the first to nth transducer elements 308-1 to 308-n include piezoelectric elements with emitting faces that are curved. For instance, the piezoelectric elements may include cylindrical shapes that emit the ultrasound signal 310 from curved surfaces. The ultrasound signal 310, in various cases, may be a dispersed signal due to the shape of the curved surfaces, wherein at least a portion of the ultrasound signal 310 is non-perpendicular and nonparallel to the housing and/or the skin (e.g., non-perpendicular and nonparallel to a blood vessel located underneath the skin and/or blood flowing in the blood vessel).

According to some examples, the first to *n*th transducer elements 308-1 to 308*-n* include non-cubic piezoelectric elements, each with at least one flat emitting side. For instance, the piezoelectric elements may have non-rectangular cross-sections, such as triangular, pentangular, or hexangular cross-sections, wherein sides of the non-rectangular cross-sections extend along the emitting sides of the piezoelectric elements. The first to *n*th transducer elements 308-1 to 308*-n*, in various cases, are arranged such that the emitting sides of the piezoelectric elements are non-perpendicular and nonparallel to the housing and/or the skin. In some cases, other sides of the piezoelectric elements are parallel to the housing and/or the skin (e.g., non-perpendicular and nonparallel to a blood vessel located underneath the skin and/or blood flowing in the blood vessel).

In some cases, the ultrasound signal 310 is angled due to the physical location of the first to *n*th transducer elements 308-1 to 308-*n*. For example, a spacer may be disposed between the first to *n*th transducer elements 308-1 to 308-*n* and the housing and/or skin that causes the first to *n*th transducer elements 308-1 to 308-*n* to be arranged in a line that is non-perpendicular and nonparallel to the housing and/or the skin (e.g., non-perpendicular and nonparallel to a blood vessel located underneath the skin and/or blood flowing in the blood vessel).

In some examples, the first to *n*th driving signals 306-1 to 306-*n* are configured to perform beamforming, such that the ultrasound signal 310 is non-perpendicular and nonparallel to the housing and/or the skin (e.g., non-perpendicular and nonparallel to a blood vessel located underneath the skin and/or blood flowing in the blood vessel). For example, the first to *n*th driving signals 306-1 to 306-*n* may correspond to respective phases that cause the ultrasound signal 310 to be angled with respect to the first to *n*th transducer elements 308-1 to 308-*n*. Various beamforming techniques are possible, such as linear array beamforming, phased array beamforming, multi-line transmission beamforming, diverging wave beamforming, synthetic aperture beamforming, active analog beamforming, passive analog beamforming, and the like. Various techniques for beamforming are described, for instance, by Demi, Appl. Sci. 2018 8(9) 1544 (2018), which is incorporated by reference herein in its entirety. In some cases, the first to *n*th driving signals 306-1 to 306-*n* are not configured to perform beamforming. For example, in various cases described herein, the ultrasound signal 310 may achieve a desired transmission direction without the first to *n*th driving signals 306-1 to 306-*n* performing beamforming. In some examples, the first to *n*th driving signals 306-1 to 306-*n* cause switching between activation of the first to *n*th transducer elements 308-1 to 308-*n*.

According to various implementations of the present disclosure, the ultrasound signal 310 is directed toward blood in a blood vessel of the subject. The ultrasound signal 310, in various cases, is reflected by the blood. The reflection of the ultrasound signal 310 may be detected by the first to nth transducer elements 308-1 to 308*-n*, or by other transducer elements (not illustrated). According to various cases, the velocity of the blood, as well as other physiological parameters, can be derived based on the reflection. In particular examples, the ultrasound signal 310 can be used to achieve pulse-wave Doppler and/or continuous wave Doppler monitoring techniques.

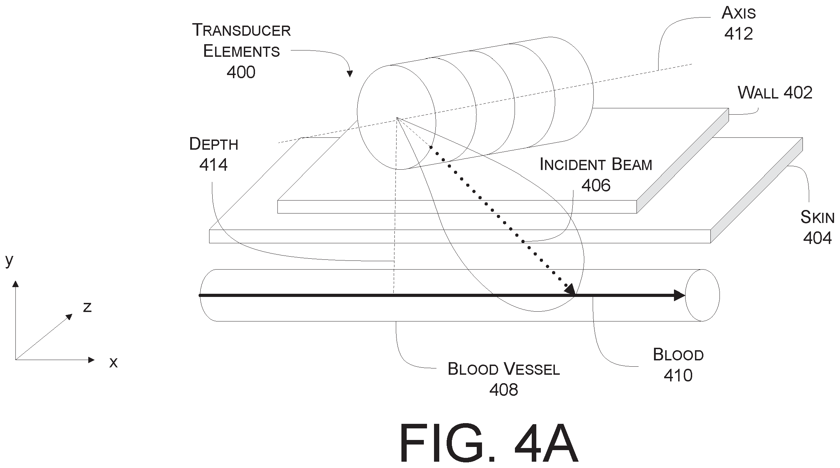

FIG. 4A illustrates an example of an array of cylindrical transducer elements 400. The transducer elements 400, for example, are examples of non-cubic transducer elements. Collectively, in some cases, the transducer elements 400 have a circular cross-section. In various cases, the transducer elements 400 are enclosed in a housing that includes at least one wall 402 configured to be disposed on skin 404 of a subject. The transducer elements 400, for example, are configured to output an incident beam 406 that is transmitted through the wall 402 of the housing and the skin 404. In some cases, the incident beam 406 includes an ultrasound lobe. A blood vessel 408, in some cases, is disposed underneath the surface of the skin 404. The blood vessel 408 is at least partially parallel to the skin 404. Blood 410 is disposed within the blood vessel 408. When the blood 410 is circulating through the body of the subject, the blood has a nonzero velocity through the blood vessel 408. In particular cases, the blood 410 may be traveling in a positive x direction through the blood vessel 408.

The transducer elements 400 are stacked along an axis 412. In various cases, the axis 412 extends in an xz direction. For instance, the axis 412 includes at least one component that extends perpendicularly to the blood vessel 408. The transducer elements 400 are separated from the blood 410 in the blood vessel 408 by a depth 414. For example, the depth 414 extends in a y direction.

In various implementations, the incident beam 406 is emitted from the curved surface of at least one of the transducer elements 400. In some cases, a selected transducer element among the transducer elements 400 emits the incident beam 406. For example, the transducer element is selected by identifying a reflection, among multiple reflections from incident beams respectively emitted by the transducer elements 400, corresponding to a Doppler shift (e.g., a frequency shift or a phase shift) with respect to the incident beams having the highest magnitude. According to various cases, the incident beams may include pulse wave (PW) or continuous wave (CW) beams, such that the Doppler shift represents PW and/or CW effects.

In various cases, the selected transducer element among the transducer elements 400 emits a cylindrical signal from the curved surface of the transducer element. A component of the cylindrical signal that intersects the blood 410 in the blood vessel 408 includes the incident beam 406. Therefore, even in cases where the blood vessel 408 and the axis 412 are parallel to an xz plane, the incident beam 406 may nevertheless be non-perpendicular to the blood 410 in the blood vessel 408. The incident beam 406 may be utilized to identify the velocity of the blood 410 using Doppler-based techniques.

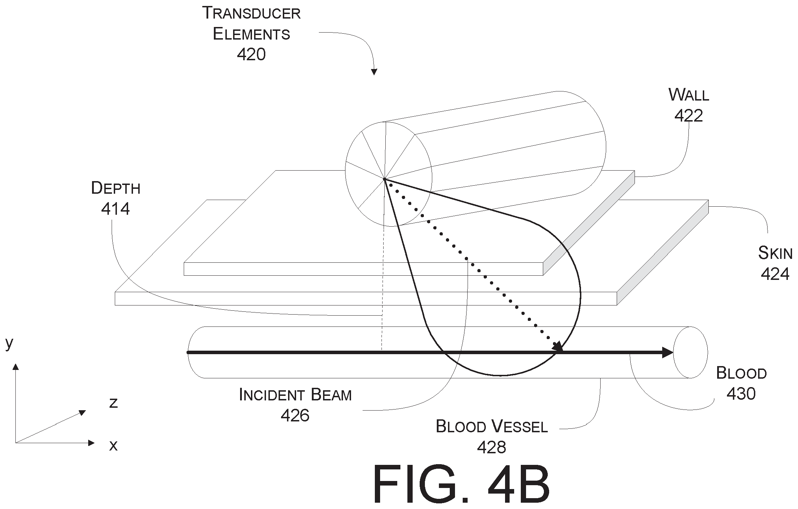

FIG. 4B illustrates an example of a cylindrical array of transducer elements 420. In various cases, the transducer elements 420 are enclosed in a housing that includes at least one wall 422 configured to be disposed on skin 424 of a subject. The transducer elements 420, for example, are examples of non-cubic transducer elements. Collectively, in some cases, the transducer elements 420 have a circular cross-section. The transducer elements 400, for example, are configured to output an incident beam 426 that is transmitted through the wall 422 of the housing and the skin 424. In some cases, the incident beam 426 includes an ultrasound lobe. A blood vessel 428, in some cases, is disposed underneath the surface of the skin 424. The blood vessel 428 is at least partially parallel to the skin 424. Blood 430 is disposed within the blood vessel 408. When the blood 430 is circulating through the body of the subject, the blood has a nonzero velocity through the blood vessel 428. In particular cases, the blood 430 may be traveling in a positive x direction through the blood vessel 428.

In various implementations, the incident beam 426 is emitted from the curved surface of at least one of the transducer elements 420. In some cases, a selected transducer element among the transducer elements 420 emits the incident beam 426. For example, the transducer element is selected by identifying a reflection, among multiple reflections from incident beams respectively emitted by the transducer elements 420, corresponding to a Doppler shift with respect to the incident beams having the highest magnitude.

In various cases, the selected transducer element among the transducer elements 420 emits a curved wavefront from the curved surface of the selected transducer element. A component of the curved waveform that intersects the blood 430 in the blood vessel 428 includes the incident beam 426. Therefore, even in cases where the blood vessel 428 and the transducer elements 420 extend along axes that are parallel to an xz plane, the incident beam 426 may nevertheless be non-perpendicular to the blood 430 in the blood vessel 428. The incident beam 426 may be utilized to identify the velocity of the blood 430 using Doppler-based techniques.

In various cases, the transducer elements 420 can be combined with a passive phase network (e.g., resistors, capacitors, and/or inductors for continuous wave (CW) or narrow band pulse wave (PW) Doppler insonification) to increase a directionality of the incident beam 426.

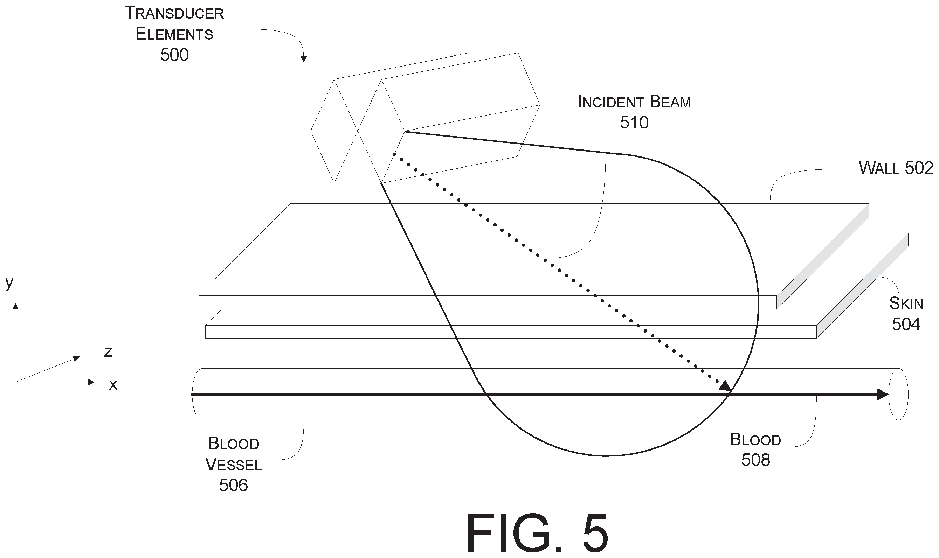

FIG. 5 illustrates an example of a transducer elements 500 that collectively form a polygonal prism. Although multiple transducer elements 500 are illustrated in FIG. 5, implementations are not so limited. For example, the transducer elements 500 may be substituted with a single transducer element having the collective shape of the transducer elements 500 illustrated in FIG. 5. The transducer elements 500, for example, are examples of non-cubic transducer elements. Collectively, in some cases, the transducer elements 500 have a polygonal cross-section. In various implementations, the polygonal cross-section has greater than four sides. The transducer elements 500 are different than the transducer elements 420 described with respect to FIG. 4B in that they are configured to emit signals from flat emitting faces, rather than curved emitting faces. For example, a cross-section of the prism includes a hexagon and the emitting side of each of the transducer elements 500 extends along a face of the hexagon. In various cases, the cross-section is defined on an xy plane, and the transducer elements 500 extend along a z direction. The transducer elements 500, in various cases, are part of a flow sensor device. A housing of the device includes a wall 502. In some examples, the wall 502 is disposed along skin 504 of a subject. A blood vessel 506 is disposed underneath the skin 504 and extends in an x direction. Blood 508 is disposed in the blood vessel 506. When the blood 508 is spontaneously circulating in the body of the subject, for instance, the blood 508 travels in a positive x direction.

According to various cases, the emitting face of one of the transducer elements 500 is parallel to the wall 502, the skin 504, and the direction in which the blood 508 is flowing through the blood vessel. However, other emitting faces of the transducer elements 500 are nonparallel to the wall 502, the skin 504, and the direction in which the blood 508 is flowing through the blood vessel 506. For instance, one of the transducer elements 500 emits an incident beam 510 that is non-perpendicular to the blood vessel 506 and the direction in which the blood 508 is flowing. In various cases, the incident beam 510 includes ultrasound. In some cases, techniques can utilize two or more transducer elements 500 that are passively phase networked together to create a beam pointing against or with the flow for narrowband (relative to the carrier) signaling.

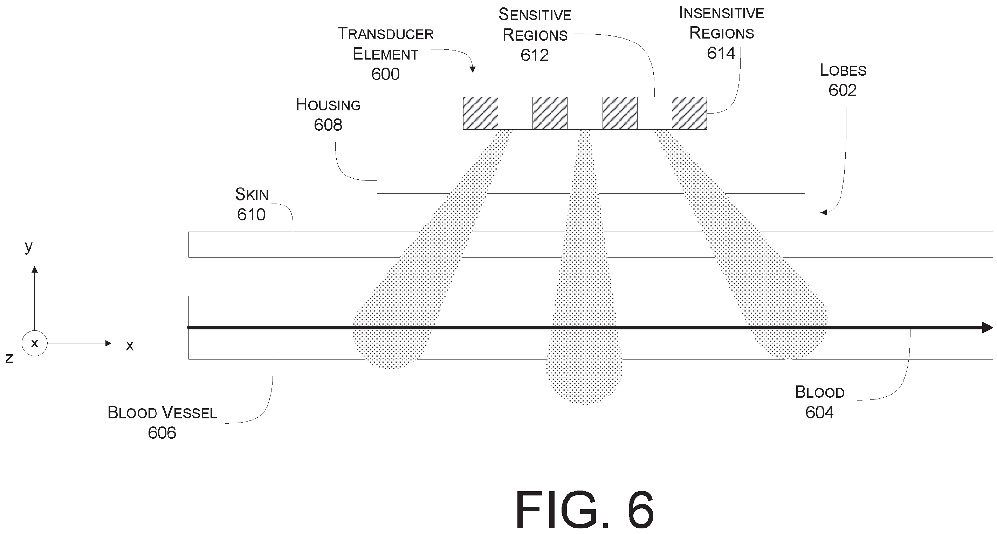

FIG. 6 illustrates an example of a planar transducer element 600 configured to generate lobes 602 of ultrasound that are transmitted in multiple directions. In various implementations, the transducer element 600 extends on an axis that is parallel to the flow of blood 604 through a blood vessel 606. The blood 604, for instance, is flowing in an x direction. The transducer element 600 is configured to transmit the lobes 602 through a wall 608 of a housing disposed against skin 610 of a subject.

The transducer element 600 communicatively coupled to a signal generator configured to output electrical signals that cause the transducer element 600 to emit the lobes 602 into the body of the subject. In various implementations, the transducer element 600 includes sensitive regions 612 and insensitive regions 614. For example, the sensitive regions 612 may include a piezoelectric material, a PMUT element, or a CMUT element; and the insensitive regions 614 may include a non-acoustic radiating material, such as a polymer, a rubber (e.g., RTV), or an air gap. The spacing of the sensitive regions 612 by the insensitive regions 614 are then used to create grating lobes 602 (e.g., spatial aliasing) so that there is sensitivity in directions in addition to the broadside direction. The widths of the insensitive regions 614 (as well as the spacing of the sensitive regions 612), as measured in wavelengths of the ultrasound, control the direction and shape of the lobes 602 in an advantageous way to maximize the Doppler signal. The length along the interdigitated sensitive regions 612 and insensitive regions 614 accrues beam strength. The width determines the beam width spanning the diameter of the blood vessel 606 that is being insonified.

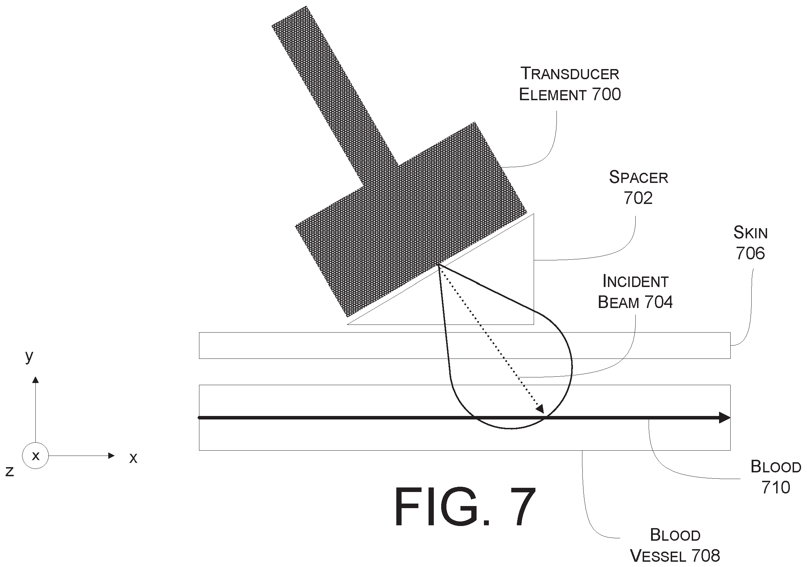

FIG. 7 illustrates an example of a transducer element 700 used with a wedge-shaped spacer 702. The transducer element 700 emits an incident beam 704 in a direction perpendicular to the emitting face of the transducer element 700. However, due to the presence of the spacer 702, the incident beam 704 travels in a direction that is non-perpendicular and nonparallel to skin 706, a blood vessel 708, and blood 710 flowing through the blood vessel 708. In some cases, the spacer 702 is a wedge or pouch that includes a material that is substantially transparent to the incident beam 704. For example, the spacer 702 includes a material that has substantially the same impedance as the transducer element 700, the skin 706, the blood vessel 708, the blood 710, or any combination thereof. In various instances, the spacer 702 includes least one of a silicone, polyvinyl alcohol (PVA), polyacrylamide (PAA), polyethylene (PE) and polymethyl-methacrylate (PMMA), polyurethane, polyvinyl chloride (PVC), or an RTV silicone. In various cases, an impedance or absorption of the material in the spacer 702 with respect to the incident beam 704 is less than a threshold impedance or absorption, such that it has limited absorption and/or reflection of the incident beam 704. Accordingly, the incident beam 704 may be substantially transmitted through the spacer 702 and into the body of the subject.

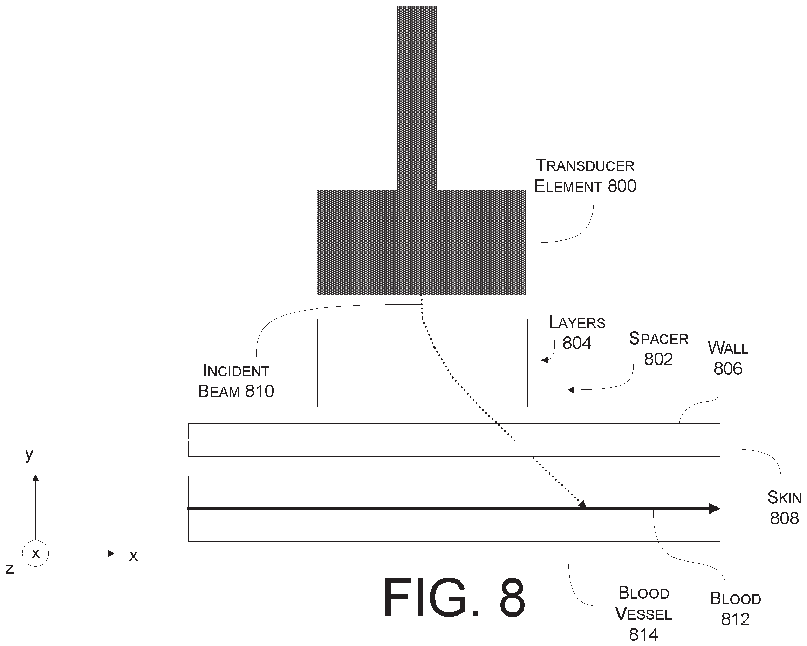

FIG. 8 illustrates an example of a transducer element 800 used with a layered spacer 802. The spacer 802, for instance, includes one or more layers 804 that are stacked in a y direction between the transducer element 800 and a wall 806 of a housing of a device that includes the transducer element 800. For example, the spacer 802 may include multiple layers that gradually transition from a similar impedance to the transducer element 800 to a similar impedance to the skin 808. The wall 806, for instance, is disposed against skin 808 of a subject. The transducer element 800 is configured to emit an incident beam 810 into the body of the subject. For example, the incident beam 810 is reflected off of blood 812 flowing through a blood vessel 814 in an x direction.

In various cases, the layers 804 include at least one layer having a first acoustic impedance and at least one layer including a second acoustic impedance. The incident beam 810, for instance, includes ultrasound. The incident beam 810 may propagate through different materials within the layers 804 at different speeds. In various cases, the layers 804 include at least one of silicone, polyvinyl alcohol (PVA), polyacrylamide (PAA), polyethylene (PE) and polymethyl-methacrylate (PMMA), polyurethane, polyvinyl chloride (PVC), or an RTV silicone. Due to the different materials within the layers 804, the layers 804 may collectively bend the incident beam 810 from a direction that is perpendicular to the flow of the blood 812 to a direction that is non-perpendicular to the flow of the blood 812. In some cases, the layers 804 have different shapes, such as wedges, stair patterns, or the like, that further bend the incident beam 810.

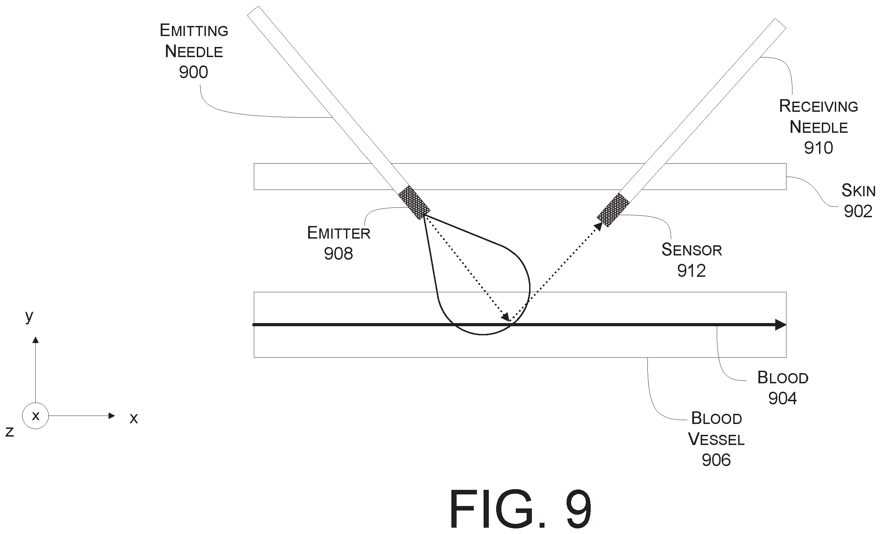

FIG. 9 illustrates an example of monitoring blood flow using needles. For example, an emitting needle 900 is configured to be inserted through skin 902 of a subject. In particular, the needle 900 is inserted through the skin 902 in a direction that is non-perpendicular and nonparallel to blood 904 flowing through a blood vessel 906 underneath the skin 902.

An emitter 908 (e.g., a transducer element) is utilized with the emitting needle 900. For example, the emitter 908 is disposed at a tip of the needle 900 inserted through the skin 902. In various cases, the emitter 908 outputs an ultrasound signal. The tip of the needle 900, for instance, includes an acoustically transparent structural material that precedes the transducer oriented to emit or receive ultrasound energy in the direction of the needle 900. In some cases, the emitter 908 includes a cylindrical element so that the needle is inserted orthogonally to the vessel.

In some examples, a receiving needle 910 is used with the emitting needle 900. The receiving needle 910, for instance, includes a sensor 912 disposed at a tip of the receiving needle 910. In some cases, the flow of the blood 904 through the blood vessel 906 is detected based on a reflection of the ultrasound signal detected by the sensor 912. The reflection, for instance, is reflected from the blood 904 in the blood vessel 906. In some cases, the emitter 908 itself acts as a sensor in addition to, or instead of, the sensor 912. For example, the emitter 908 includes an ultrasound transducer configured to output and detect ultrasound.

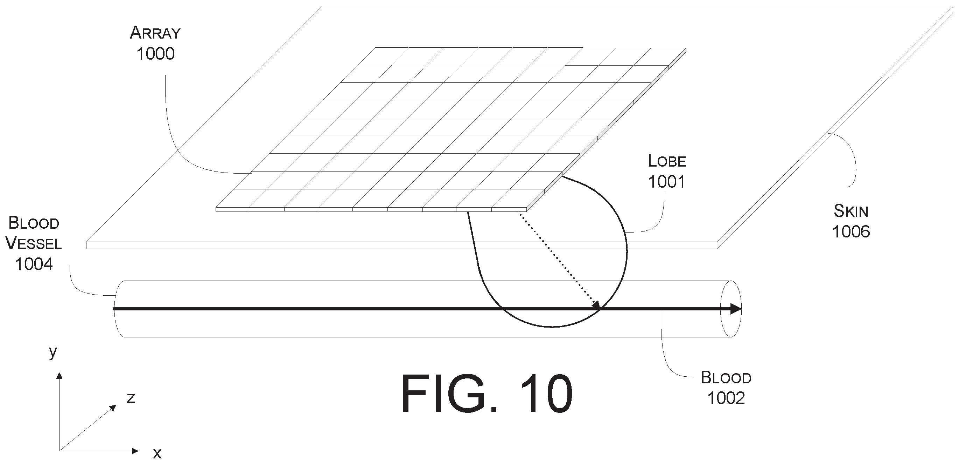

FIG. 10 illustrates an example of a 2D transducer element array 1000 that implements a phase delay (e.g., using analog or digital means) among the transducer elements. For example, the phase delay is implemented by a signal generator. Optionally, the signal generator includes at least one preamplifier. Due to the phase delay, the array 1000 collectively outputs an ultrasound lobe 1001 that is non-perpendicular and nonparallel to blood 1002 flowing through a blood vessel 1004 of a subject on whose skin 1006 a device including the array 1000 is disposed. For instance, a center of the lobe 1001 is nonparallel and non-perpendicular to an x direction, but nevertheless intersects the direction of the blood 1002 flowing through the blood vessel 1004. In various cases, the array 1000 can also be embedded in an acoustically conducting material. Based on a reflection of the lobe 1001 from the blood 1002, the velocity of the blood 1002 can be detected.

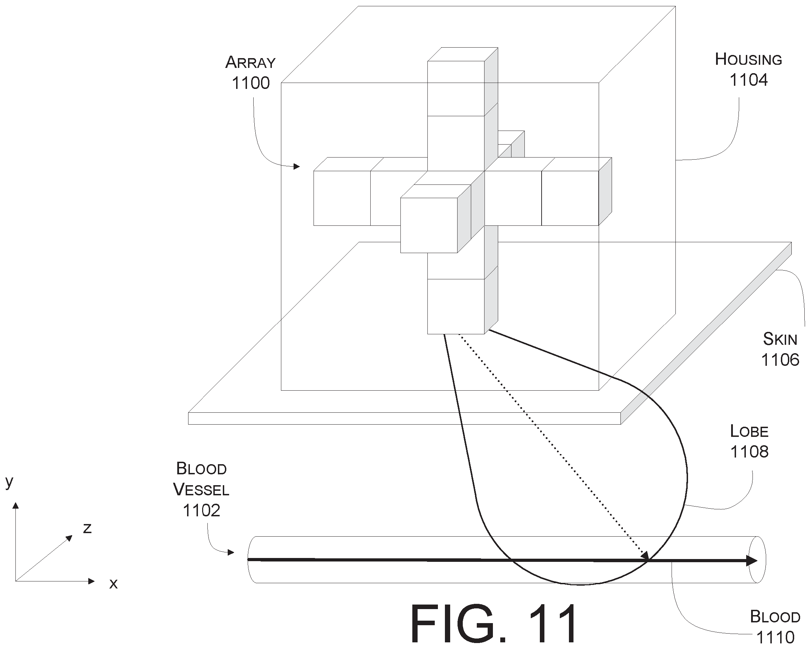

FIG. 11 illustrates an example of a 3D transducer element array 1100. The array 1100 includes a 3D surface of elements at have an acoustic path to a blood vessel 1102 vessel when a housing 1104 of a device including the array 1100 is applied to skin 1106 of a subject. Accordingly, when a signal generator implements a phase delay of driving signals to the elements of the array 1100, the center of a lobe 1108 of ultrasound emitted by the array 1100 is non-perpendicular and nonparallel to the flow of blood 1110 through the blood vessel 1102. In some cases, an initial orientation between the housing 1104 and the blood vessel 1102 is irrelevant, as either the best element is selected or the lobe 1108 formed from the elements (e.g., using analog or digital means) is created to sense the signal representing the greatest Doppler shift.

In some cases, a flow monitor is configured to emit three ultrasound signals (e.g., lobes) that are respectively centered in three linearly independent directions. The flow monitor may include any combination of the transducer elements, emitters, transmitters, or arrays described above with reference to FIGS. 4A to FIG. 11, in order to achieve the linear independence of the ultrasound signals. In various cases, a rescuer may place the flow monitor on the skin of a subject, without specifically positioning the flow monitor in any specific direction or orientation. The flow monitor, for instance, emits the three ultrasound signals after being placed on the subject. In some cases, the ultrasound signals are emitted sequentially. Due to the linear independence of the ultrasound signals, at least one of the ultrasound signals may be emitted in a direction that is nonparallel and nonperpendicular to a blood vessel of interest. In various cases, the flow monitor may detect the flow of blood through the blood vessel of interest based on the ultrasound signal associated with the most significant Doppler shift due to the blood in the blood vessel.

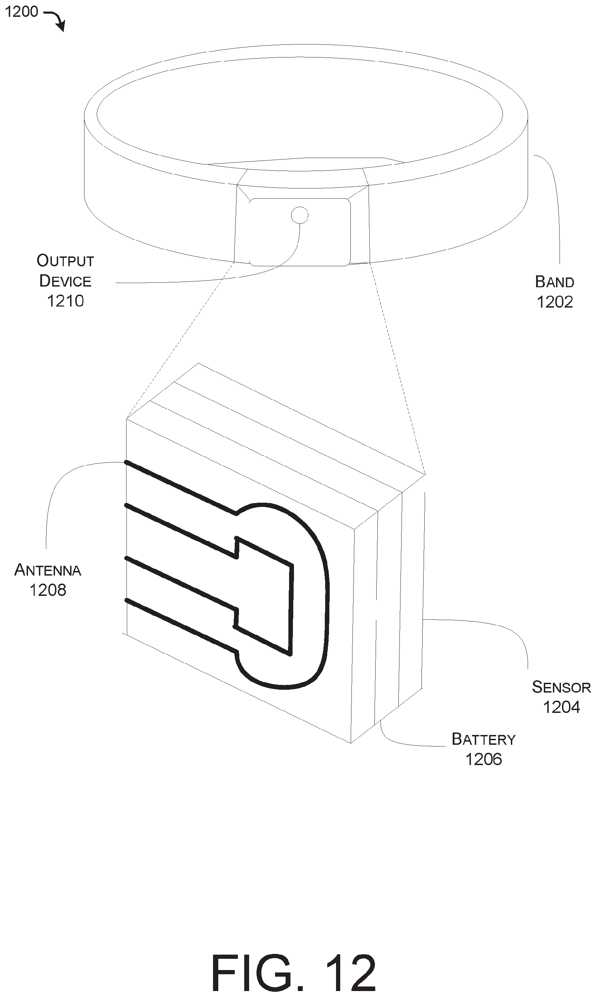

FIG. 12 illustrates the structure of an example flow monitor 1200. In some cases, the flow monitor 1200 is a patch that can be strapped to a subject or adhered to the skin of the subject. For instance, the flow monitor 1200 includes a band 1202 configured to be disposed around an appendage of the subject. According to some examples, the band 1202 is configured to be disposed around a chest, abdomen, head, or neck of the subject.

In various implementations, the flow monitor 1200 includes a sensor 1204 configured to detect a physiological parameter indicative of blood flow through a subject. In some cases, the sensor 1204 includes a microphone configured to detect a sound generated by the body of the subject. In some examples, the sensor 1204 includes an ultrasound transducer configured to detect the velocity of blood flowing through at least one blood vessel of the subject and/or through the heart of the subject. In some examples, the sensor 1204 includes an ultrasound transducer configured to detect heart wall motion. In some cases, the sensor 1204 includes at least one transmitter and/or at least one receiver. In some examples, the sensor 1204 includes one or more accelerometers.

The sensor 1204, for instance, is powered by a battery 1206. In some cases, the battery 1206 is disposable. In some examples, the battery 1206 is rechargeable.

The flow monitor 1200, in various cases, further includes an antenna 1208. The antenna 1208, in various implementations, is configured to transmit and/or receive communication signals from an external device. In some cases, the antenna 1208 enables the flow monitor 1200 to communicate with another flow monitor or another type of medical device (e.g., an AED or monitor-defibrillator). For instance, the flow monitor 1200 is configured to transmit, to an external device, an indication of the physiological parameter detected by the sensor 1204. The antenna 1208, in various cases, is powered by the battery 1206.

According to various examples, the sensor 1204, the battery 1206, and the antenna 1208 are arranged in respective layers of the flow monitor 1200. For example, each layer may correspond to a circuit board (e.g., a PCB) that accommodates a respective component. In various cases, the layer corresponding to the sensor 1204 is configured to be adjacent to the subject when the flow monitor 1200 is disposed on the subject. The layer corresponding to the antenna 1208, for example, faces away from the subject when the flow monitor 1200 is disposed on the subject. Accordingly, the body of the subject may be prevented from interfering with wireless signals transmitted and/or received by the antenna 1208.

In some examples, the flow monitor 1200 further includes an output device 1210. The output device 1210 is configured to output a signal to a user. In some cases, the signal is based on the physiological parameter detected by the sensor 1204. For instance, the output device 1210 includes a light that is activated when a blood velocity detected by the sensor 1204 remains below a threshold blood velocity for greater than a threshold period of time. Various signals that are output may include alerts that prompt a rescuer to manually attend to the subject that the flow monitor 1200 is monitoring.

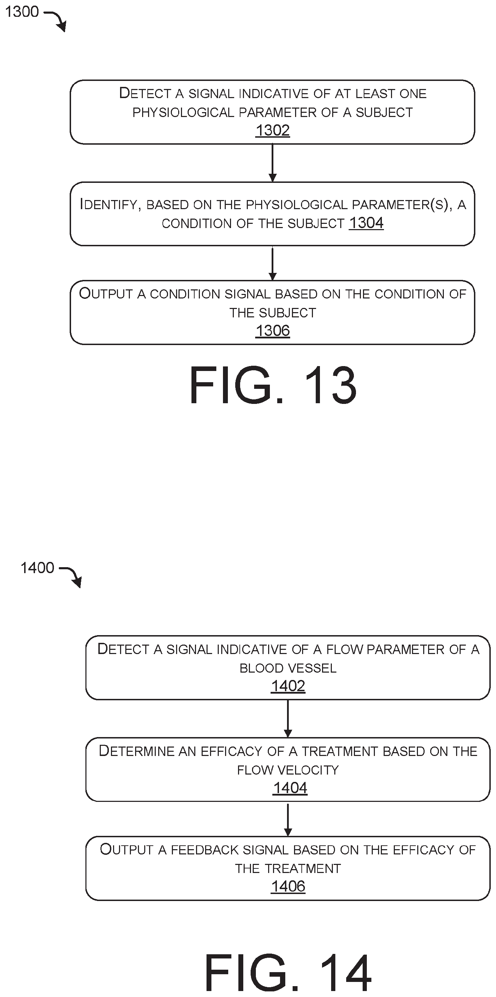

FIG. 13 illustrates an example method 1300 for identifying a condition of a subject. In various cases, the method 1300 is performed by an entity, such as a flow monitor, medical device, computing device, at least one processor, or a combination thereof. At 1302, the entity receives a signal indicative of at least one physiological parameter of a subject. The entity may detect the at least one physiological parameter of the subject. In some cases, the physiological parameter(s) include a flow velocity of blood flowing through at least one blood vessel. At 1304, the entity identifies, based on the physiological parameter(s), a condition of the subject. For instance, the entity detects a net blood flow to or from an area of the body associated with the blood vessel. At 1306, the entity outputs a condition signal based on the condition of the subject. For example, the entity displays an indication of the condition, audibly outputs an indication of the condition, or transmits (e.g., to an external device) a signal encoded with an indication of the condition.

FIG. 14 illustrates an example method 1400 for providing feedback on a treatment performed on the subject. In various cases, the method 1400 is performed by an entity, such as a flow monitor, medical device, computing device, at least one processor, or a combination thereof. At 1402, the entity receives a signal indicative of a flow parameter of a blood vessel. For example, the entity receives a signal indicative of a flow rate through a blood vessel of the subject. The entity may detect the flow parameter of the blood vessel. At 1404, the entity determines an efficacy of a treatment based on the flow velocity. For example, the entity determines an efficacy of chest compressions administered to the subject based on the flow parameter. At 1406, the entity outputs a feedback signal based on the efficacy of the treatment. In some cases, the feedback signal includes an instruction to change a parameter of the chest compressions administered to the subject.

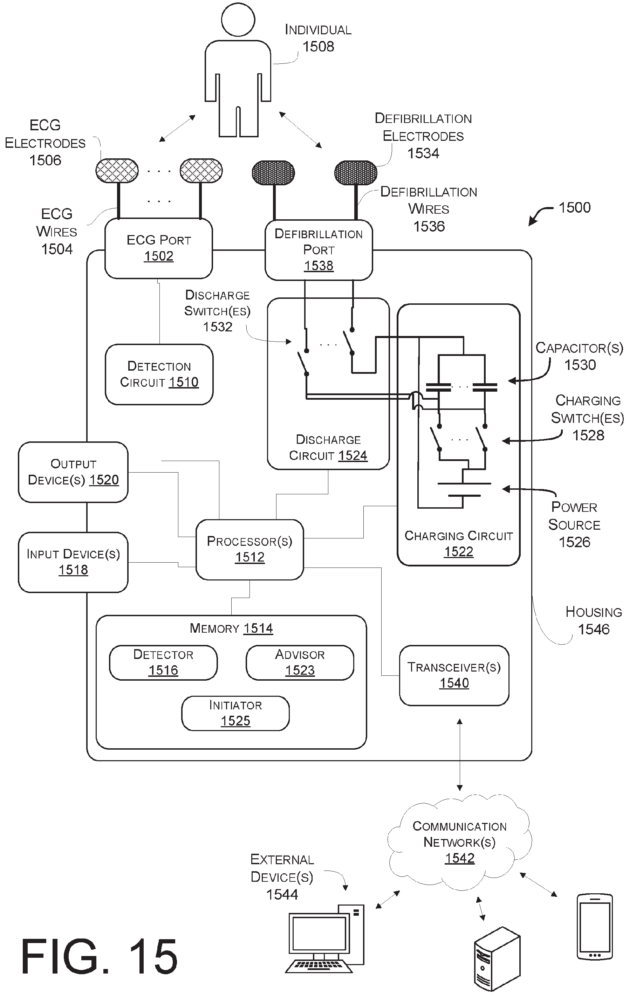

FIG. 15 illustrates an example of an external defibrillator 1500 configured to perform various functions described herein. For example, the external defibrillator 1500 is the monitor-defibrillator 128 described above with reference to FIG. 1.

The external defibrillator 1500 includes an electrocardiogram (ECG) port 1502 connected to multiple ECG wires 1504. In some cases, the ECG wires 1504 are removeable from the ECG port 1502. For instance, the ECG wires 1504 are plugged into the ECG port 1502. The ECG wires 1504 are connected to ECG electrodes 1506, respectively. In various implementations, the ECG electrodes 1506 are disposed on different locations on an individual 1508. A detection circuit 1510 is configured to detect relative voltages between the ECG electrodes 1506. These voltages are indicative of the electrical activity of the heart of the individual 1508.

In various implementations, the ECG electrodes 1506 are in contact with the different locations on the skin of the individual 1508. In some examples, a first one of the ECG electrodes 1506 is placed on the skin between the heart and right arm of the individual 1508, a second one of the ECG electrodes 1506 is placed on the skin between the heart and left arm of the individual 1508, and a third one of the ECG electrodes 1506 is placed on the skin between the heart and a leg (either the left leg or the right leg) of the individual 1508. In these examples, the detection circuit 1510 is configured to measure the relative voltages between the first, second, and third ECG electrodes 1506. Respective pairings of the ECG electrodes 1506 are referred to as "leads," and the voltages between the pairs of ECG electrodes 1506 are known as "lead voltages." In some examples, more than three ECG electrodes 1506 are included, such that 5-lead or 12-lead ECG signals are detected by the detection circuit 1510.

The detection circuit 1510 includes at least one analog circuit, at least one digital circuit, or a combination thereof. The detection circuit 1510 receives the analog electrical signals from the ECG electrodes 1506, via the ECG port 1502 and the ECG wires 1504. In some cases, the detection circuit 1510 includes one or more analog filters configured to filter noise and/or artifact from the electrical signals. The detection circuit 1510 includes an analog-to-digital converter (ADC) in various examples. The detection circuit 1510 generates a digital signal indicative of the analog electrical signals from the ECG electrodes 1506. This digital signal can be referred to as an "ECG signal" or an "ECG."

In some cases, the detection circuit 1510 further detects an electrical impedance between at least one pair of the ECG electrodes 1506. For example, the detection circuit 1510 includes, or otherwise controls, a power source that applies a known voltage (or current) across a pair of the ECG electrodes 1506 and detects a resultant current (or voltage) between the pair of the ECG electrodes 1506. The impedance is generated based on the applied signal (voltage or current) and the resultant signal (current or voltage). In various cases, the impedance corresponds to respiration of the individual 1508, chest compressions performed on the individual 1508, and other physiological states of the individual 1508. In various examples, the detection circuit 1510 includes one or more analog filters configured to filter noise and/or artifact from the resultant signal. The detection circuit 1510 generates a digital signal indicative of the impedance using an ADC. This digital signal can be referred to as an "impedance signal" or an "impedance."

The detection circuit 1510 provides the ECG signal and/or the impedance signal one or more processors 1512 in the external defibrillator 1500. In some implementations, the processor(s) 1512 includes a central processing unit (CPU), a graphics processing unit (GPU), digital signal processing unit (DPU), other processing unit or component known in the art, or any combination thereof.

The processor(s) 1512 is operably connected to memory 1514. In various implementations, the memory 1514 is volatile (such as random access memory (RAM)), non-volatile (such as read only memory (ROM), flash memory, etc.) or some combination of the two. The memory 1514 stores instructions that, when executed by the processor(s) 1512, causes the processor(s) 1512 to perform various operations. In various examples, the memory 1514 stores methods, threads, processes, applications, objects, modules, any other sort of executable instruction, or a combination thereof. In some cases, the memory 1514 stores files, databases, or a combination thereof. In some examples, the memory 1514 includes, but is not limited to, RAM, ROM, electrically erasable programmable read-only memory (EEPROM), flash memory, or any other memory technology. In some examples, the memory 1514 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 1512 and/or the external defibrillator 1500. In some cases, the memory 1514 at least temporarily stores the ECG signal and/or the impedance signal.

In various examples, the memory 1514 includes a detector 1516, which causes the processor(s) 1512 to determine, based on the ECG signal and/or the impedance signal, whether the individual 1508 is exhibiting a particular heart rhythm. For instance, the processor(s) 1512 determines whether the individual 1508 is experiencing a shockable rhythm that is treatable by defibrillation. Examples of shockable rhythms include ventricular fibrillation (VF) and ventricular tachycardia (VT). In some examples, the processor(s) 1512 determines whether any of a variety of different rhythms (e.g., asystole, sinus rhythm, atrial fibrillation (AF), etc.) are present in the ECG signal.

The processor(s) 1512 is operably connected to one or more input devices 1518 and one or more output devices 1520. Collectively, the input device(s) 1518 and the output device(s) 1520 function as an interface between a user and the defibrillator 1500. The input device(s) 1518 is configured to receive an input from a user and includes at least one of a switch, a keypad, a cursor control, a touch-sensitive display, a voice input device (e.g., a microphone), a haptic feedback device (e.g., a gyroscope), or any combination thereof. The output device(s) 1520 includes at least one of a display, a speaker, a haptic output device, a printer, a light indicator such as an LED, or any combination thereof. In various examples, the processor(s) 1512 causes a display among the input device(s) 1518 to visually output a waveform of the ECG signal and/or the impedance signal. In some implementations, the input device(s) 1518 includes one or more touch sensors, the output device(s) 1520 includes a display screen, and the touch sensor(s) are integrated with the display screen. Thus, in some cases, the external defibrillator 1500 includes a touchscreen configured to receive user input signal(s) and visually output physiological parameters, such as the ECG signal and/or the impedance signal.

In some examples, the memory 1514 includes an advisor 1523, which, when executed by the processor(s) 1512, causes the processor(s) 1512 to generate advice and/or control the output device(s) 1520 to output the advice to a user (e.g., a rescuer). In some examples, the processor(s) 1512 provides, or causes the output device(s) 1520 to provide, an instruction to perform CPR on the individual 1508. In some cases, the processor(s) 1512 evaluates, based on the ECG signal, the impedance signal, or other physiological parameters, CPR being performed on the individual 1508 and causes the output device(s) 1520 to provide feedback about the CPR in the instruction. According to some examples, the processor(s) 1512, upon identifying that a shockable rhythm is present in the ECG signal, causes the output device(s) 1520 to output an instruction and/or recommendation to administer a defibrillation shock to the individual 1508.

The memory 1514 also includes an initiator 1525 which is configured to, when executed by the processor(s) 1512, cause the processor(s) 1512 to control other elements of the external defibrillator 1500 in order to administer a defibrillation shock to the individual 1508. In some examples, the processor(s) 1512 executing the discharge circuit 1524 is configured to selectively cause the administration of the defibrillation shock based on determining that the individual 1508 is exhibiting the shockable rhythm and/or based on an input from a user (received, e.g., by the input device(s) 1518. In some cases, the processor(s) 1512 causes the defibrillation shock to be output at a particular time, which is determined by the processor(s) 1512 based on the ECG signal and/or the impedance signal.

The processor(s) 1512 is operably connected to a charging circuit 1522 and a discharge circuit 1524. In various implementations, the charging circuit 1522 includes a power source 1526, one or more charging switches 1528, and one or more capacitors 1530. The power source 1526 includes, for instance, a battery. The processor(s) 1512 initiates a defibrillation shock by causing the power source 1526 to charge at least one capacitor among the capacitor(s) 1530. For example, the processor(s) 1512 activates at least one of the charging switch(es) 1528 in the charging circuit 1522 to complete a first circuit connecting the power source 1526 and the capacitor to be charged. Then, the processor(s) 1512 causes the discharge circuit 1524 to discharge energy stored in the charged capacitor across a pair of defibrillation electrodes 1534, which are in contact with the individual 1508. For example, the processor(s) 1512 deactivates the charging switch(es) 1528 completing the first circuit between the capacitor(s) 1530 and the power source 1526, and activates one or more discharge switches 1532 completing a second circuit connecting the charged capacitor 1530 and at least a portion of the individual 1508 disposed between defibrillation electrodes 1534.

The energy is discharged from the defibrillation electrodes 1534 in the form of a defibrillation shock. For example, the defibrillation electrodes 1534 are connected to the skin of the individual 1508 and located at positions on different sides of the heart of the individual 1508, such that the defibrillation shock is applied across the heart of the individual 1508. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 1532 are controlled by the processor(s) 1512, for example. In various implementations, the defibrillation electrodes 1534 are connected to defibrillation wires 1536. The defibrillation wires 1536 are connected to a defibrillation port 1538, in implementations. According to various examples, the defibrillation wires 1536 are removable from the defibrillation port 1538. For example, the defibrillation wires 1536 are plugged into the defibrillation port 1538.

In various implementations, the processor(s) 1512 is operably connected to one or more transceivers 1540 that transmit and/or receive data over one or more communication networks 1542. For example, the transceiver(s) 1540 includes a network interface card (NIC), a network adapter, a local area network (LAN) adapter, or a physical, virtual, or logical address to connect to the various external devices and/or systems. In various examples, the transceiver(s) 1540 includes any sort of wireless transceivers capable of engaging in wireless communication (e.g., radio frequency (RF) communication). For example, the communication network(s) 1542 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 1540 includes other wireless modems, such as a modem for engaging in WI-FI^{®}, WIGIG^{®}, WIMAX^{®}, BLUETOOTH^{®}, or infrared communication over the communication network(s) 1542.

The defibrillator 1500 is configured to transmit and/or receive data (e.g., ECG data, impedance data, data indicative of one or more detected heart rhythms of the individual 1508, data indicative of one or more defibrillation shocks administered to the individual 1508, etc.) with one or more external devices 1544 via the communication network(s) 1542. The external devices 1544 include, for instance, mobile devices (e.g., mobile phones, smart watches, etc.), Internet of Things (IoT) devices, medical devices (e.g., a flow monitor, such as the flow monitor 102), computers (e.g., laptop devices, servers, etc.), or any other type of computing device configured to communicate over the communication network(s) 1542. In some examples, the external device(s) 1544 is located remotely from the defibrillator 1500, such as at a remote clinical environment (e.g., a hospital). According to various implementations, the processor(s) 1512 causes the transceiver(s) 1540 to transmit data to the external device(s) 1544. In some cases, the transceiver(s) 1540 receives data from the external device(s) 1544 and the transceiver(s) 1540 provide the received data to the processor(s) 1512 for further analysis.

In various implementations, the external defibrillator 1500 also includes a housing 1546 that at least partially encloses other elements of the external defibrillator 1500. For example, the housing 1546 encloses the detection circuit 1510, the processor(s) 1512, the memory 1514, the charging circuit 1522, the transceiver(s) 1540, or any combination thereof. In some cases, the input device(s) 1518 and output device(s) 1520 extend from an interior space at least partially surrounded by the housing 1546 through a wall of the housing 1546. In various examples, the housing 1546 acts as a barrier to moisture, electrical interference, and/or dust, thereby protecting various components in the external defibrillator 1500 from damage.

In some implementations, the external defibrillator 1500 is an automated external defibrillator (AED) operated by an untrained user (e.g., a bystander, layperson, etc.) and can be operated in an automatic mode. In automatic mode, the processor(s) 1512 automatically identifies a rhythm in the ECG signal, makes a decision whether to administer a defibrillation shock, charges the capacitor(s) 1530, discharges the capacitor(s) 1530, or any combination thereof. In some cases, the processor(s) 1512 controls the output device(s) 1520 to output (e.g., display) a simplified user interface to the untrained user. For example, the processor(s) 1512 refrains from causing the output device(s) 1520 to display a waveform of the ECG signal and/or the impedance signal to the untrained user, in order to simplify operation of the external defibrillator 1500.

In some examples, the external defibrillator 1500 is a monitor-defibrillator utilized by a trained user (e.g., a clinician, an emergency responder, etc.) and can be operated in a manual mode or the automatic mode. When the external defibrillator 1500 operates in manual mode, the processor(s) 1512 cause the output device(s) 1520 to display a variety of information that may be relevant to the trained user, such as waveforms indicating the ECG data and/or impedance data, notifications about detected heart rhythms, and the like.

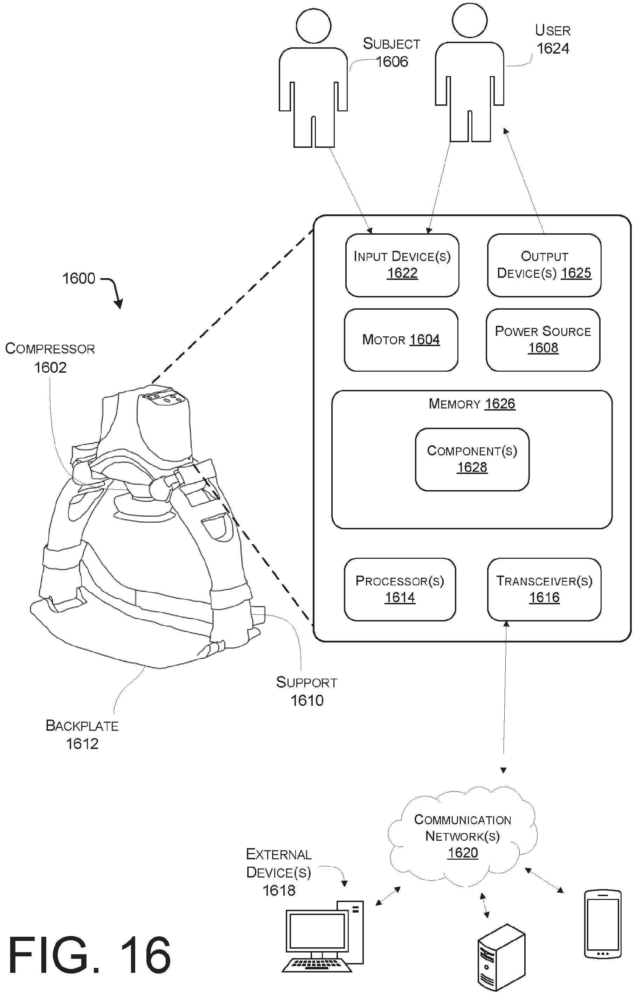

FIG. 16 illustrates a chest compression device 1600 configured to perform various functions described herein. For example, the chest compression device 1600 is the mechanical chest compression device 126 described with reference to FIG. 1.

In various implementations, the chest compression device 1600 includes a compressor 1602 that is operatively coupled to a motor 1604. The compressor 1602 is configured to physically administer a force to the chest of a subject 1606 that compresses the chest of the subject 1606. In some examples, the compressor 1602 includes at least one piston that periodically moves between two positions (e.g., a compressed position and a release position) at a compression frequency. For example, when the piston is positioned on the chest of the subject 1606, the piston compresses the chest when the piston is moved into the compressed position. A suction cup may be positioned on a tip of the piston, such that the suction cup contacts the chest of the subject 1606 during operation. In various cases, the compressor 1602 includes a band that periodically tightens to a first tension and loosens to a second tension at a compression frequency. For instance, when the band is disposed around the chest of the subject 1606, the band compresses the chest when the band tightens.

The motor 1604 is configured to convert electrical energy stored in a power source 1608 into mechanical energy that moves and/or tightens the compressor 1602, thereby causing the compressor 1602 to administer the force to the chest of the subject 1606. In various implementations, the power source 1608 is portable. For instance, the power source 1608 includes at least one rechargeable (e.g., lithium-ion) battery. In some cases, the power source 1608 supplies electrical energy to one or more elements of the chest compression device 1600 described herein.

In various cases, the chest compression device 1600 includes a support 1610 that is physically coupled to the compressor 1602, such that the compressor 1602 maintains a position relative to the subject 1606 during operation. In some implementations, the support 1610 is physically coupled to a backplate 1612, cot, or other external structure with a fixed position relative to the subject 1606. According to some cases, the support 1610 is physically coupled to a portion of the subject 1606, such as wrists of the subject 1606.

The operation of the chest compression device 1600 may be controlled by at least one processor 1614. In various implementations, the motor 1604 is communicatively coupled to the processor(s) 1614. Specifically, the processor(s) 1614 is configured to output a control signal to the motor 1604 that causes the motor 1604 to actuate or otherwise adjust the compressor 1602. For instance, the motor 1604 causes the compressor 1602 to administer the compressions to the subject 1606 based on the control signal. In some cases, the control signal indicates one or more treatment parameters of the compressions. Examples of treatment parameters include a frequency, timing, depth, force, position, velocity, and acceleration of the compressor 1602 administering the compressions. According to various cases, the control signal causes the motor 1604 to cease compressions, such as after ROSC has been detected.

In various implementations, the chest compression device 1600 includes at least one transceiver 1616 configured to communicate with at least one external device 1618 over one or more communication networks 1620. Any wired and/or wireless communication network described herein can be included in the communication network(s) 1620 illustrated in FIG. 16. The external device(s) 1618, for example, includes at least one of a flow monitor (e.g., the flow monitor 102), a monitor-defibrillator, an AED, an ECMO device, a ventilation device, a subject monitor, a mobile phone, a server, or a computing device. In some implementations, the transceiver(s) 1616 is configured to communicate with the external device(s) 1618 by transmitting and/or receiving signals wirelessly. For example, the transceiver(s) 1616 includes a NIC, a network adapter, a LAN adapter, or a physical, virtual, or logical address to connect to the various external devices and/or systems. In various examples, the transceiver(s) 1616 includes any sort of wireless transceivers capable of engaging in wireless communication (e.g., RF communication). For example, the communication network(s) 1620 includes one or more wireless networks that include a 3GPP network, such as an LTE RAN (e.g., over one or more LTE bands), an NR RAN (e.g., over one or more NR bands), or a combination thereof. In some cases, the transceiver(s) 1616 includes other wireless modems, such as a modem for engaging in WI-FI^{®}, WIGIG^{®}, WIMAX^{®}, BLUETOOTH^{®}, or infrared communication over the communication network(s) 1620. The signals, in various cases, encode data in the form of data packets, datagrams, or the like. In some cases, the signals are transmitted as compressions are being administered by the chest compression device 1600 (e.g., for real-time feedback by the external device(s) 1618), after compressions are administered by the chest compression device 1600 (e.g., for post-event review at the external device 1618), or a combination thereof.

In various cases, the processor(s) 1614 generates the control signal based on data encoded in the signals received from the external device(s) 1618. For instance, the signals include an instruction to initiate the compressions, and the processor(s) 1614 instructs the motor 1604 to begin actuating the compressor 1602 in accordance with the signals.

In some cases, the chest compression device 1600 includes at least one input device 1622. In various examples, the input device(s) 1622 is configured to receive an input signal from a user 1624, who may be a rescuer treating the subject 1606. Examples of the input device(s) 1622 include, for instance, a switch, a keypad, a cursor control, a touch-sensitive display, a voice input device (e.g., a microphone), a haptic feedback device (e.g., a gyroscope), or any combination thereof. In various implementations, the processor(s) 1614 generate the control signal based on the input signal. For instance, the processor(s) 1614 generate the control signal to adjust a frequency of the compressions based on the chest compression device 1600 detecting a selection by the user 1624 of a user interface element displayed on a touchscreen or detecting the user 1624 pressing a button integrated with an external housing of the chest compression device 1600.

According to some examples, the input device(s) 1622 include one or more sensors. The sensor(s), for example, is configured to detect a physiological parameter of the subject 1606. In some implementations, the sensor(s) is configured to detect a state parameter of the chest compression device 1600, such as a position of the compressor 1602 with respect to the subject 1606 or the backplate 1612, a force administered by the compressor 1602 on the subject 1606, a force administered onto the backplate 1612 by the body of the subject 1606 during a compression, or the like. According to some implementations, the signals transmitted by the transceiver(s) 1616 indicate the physiological parameter(s) and/or the state parameter(s).

The chest compression device 1600 further includes at least one output device 1625, in various implementations. Examples of the output device(s) 1625 include, for instance, least one of a display (e.g., a projector, an LED screen, etc.), a speaker, a haptic output device, a printer, a light source such as an LED, or any combination thereof. In some implementations, the output device(s) 1625 include a screen configured to display various parameters detected by and/or reported to the chest compression device 1600, a charge level of the power source 1608, a timer indicating a time since compressions were initiated or paused, and other relevant information.

The chest compression device 1600 further includes memory 1626. In various implementations, the memory 1626 is volatile (such as RAM), non-volatile (such as ROM, flash memory, etc.) or some combination of the two. The memory 1626 stores instructions that, when executed by the processor(s) 1614, causes the processor(s) 1614 to perform various operations. In various examples, the memory 1626 stores methods, threads, processes, applications, objects, modules, any other sort of executable instruction, or a combination thereof. In some cases, the memory 1626 stores files, databases, or a combination thereof. In some examples, the memory 1626 includes, but is not limited to, RAM, ROM, 16PROM, flash memory, or any other memory technology. In some examples, the memory 1626 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 1626 stores instructions, programs, threads, objects, data, or any combination thereof, that cause the processor(s) 1614 to perform various functions. In various cases, the memory 1626 stores one or more parameters that are detected by the chest compression device 1600 and/or reported to the chest compression device 1600.

In implementations of the present disclosure, the memory 1626 also stores one or more components 1628. The component(s) 1628 include programs, instructions, files, databases, models, or any other type of data that causes the device 1600 to perform any of the functions described herein.

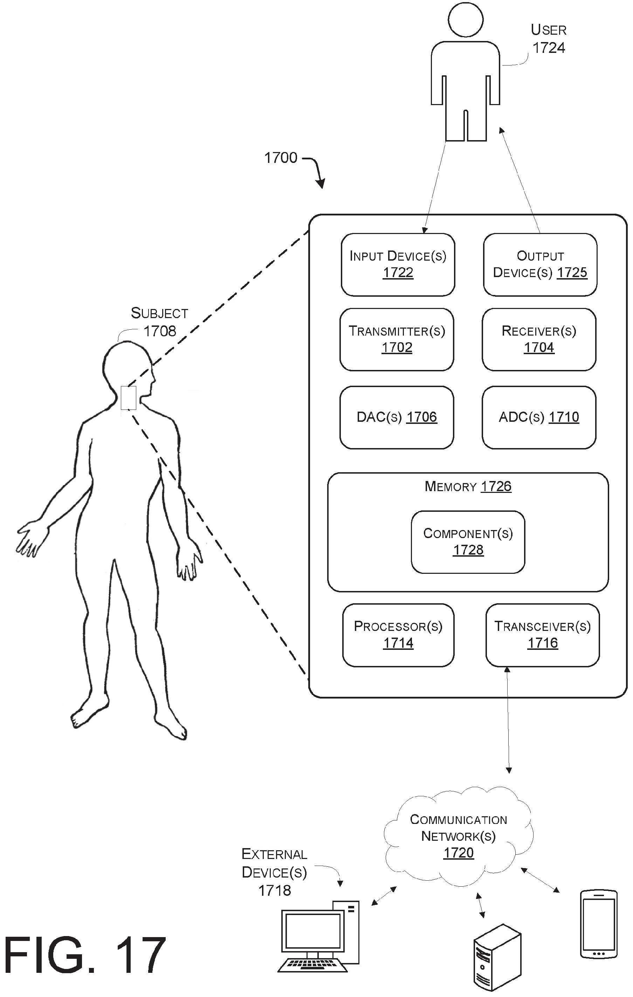

FIG. 17 illustrates a flow monitor 1700 configured to perform various functions described herein. For example, the flow monitor 1700 is the flow monitor 102 described above.

In various implementations, the flow monitor 1700 includes one or more transmitters 1702, one or more receivers 1704, one or more DACs 1706, and one or more digital to analog converters 1710. The transmitter(s) 1702 is configured to emit an incident beam into a subject 1708. The incident beam includes infrared light and/or ultrasound. For instance, the transmitter(s) 1702 include one or more light sources (e.g., one or more LEDs) or one or more piezoelectric crystals, MEMS transducers, or other electro-mechanical conversion device or material. In some examples, the flow monitor 1700 is positioned such that the incident beam is transmitted through a window in the skull of the subject 1708, such as a temple, an orbital cavity, a nasal cavity, or an ear canal. In some cases, the transmitter(s) 1702 emits one or more incident beams toward an artery supplying blood to a portion of the body of the subject 1708 and a vein transporting blood out of the portion of the body of the subject 1708.

In various implementations, the incident beam is reflected or otherwise scattered by blood in a blood vessel of the subject 1708. The receiver(s) 1704 is configured to detect within a return beam that includes signals from the reflection or scatter from the blood in the blood vessel. For example, the receiver(s) 1704 include one or more light sensors or one or more piezoelectric crystals, MEMS transducers, or other electro-mechanical conversion device or material. In various cases, the flow monitor 1700 includes one or more transceivers that embody the transmitter(s) 1702 and receiver(s) 1704.

The operation of the flow monitor 1700 may be controlled by at least one processor 1714. The processor(s) 1714 are communicatively coupled to the DAC(s) 1706 and the ADC(s) 1710. The DAC(s) 1706 is configured to convert digital signals output by the processor(s) 1714 into analog signals, such as analog signals that induce the transmitter(s) 1702 to emit the incident beam. The ADC(S) 1710 is configured to convert analog signals generated by the receiver(s) 1704 (e.g., induced by detecting the return beam) into digital signals that are received by the processor(s) 1714. The processor(s) 1714 is configured to control the transmitter(s) 1702 and to analyze the signals detected by the receiver(s) 1704. The processor(s) 1714, for instance, analyze the digital signals from the ADC(s) 1710, which are indicative of the return beam detected by the receiver(s) 1704, in order to determine a flow velocity of the blood through the blood vessel. In some examples, the flow monitor 1700 includes one or more switches (e.g., MOSFETs) connected to an array of transmitters within the transmitter(s) 1702 that respectively connect the transmitter(s) 1702 between one or more power rails (e.g., negative and/or positive power rails) and ground. The processor(s) 1714, for instance, cause the switch(es) to connect each transmitter among the transmitter(s) 1702 between ground and the power rail(s) in a periodic waveform, which causes the transmitter(s) 1702 to selectively output incident beam(s) in accordance with the periodic waveform. In some cases, the waveform has a three half-cycle waveform and goes from a positive voltage, to a negative voltage, to a positive voltage, then to ground. In some cases, more than one positive power rail or more than one negative power rail is used for the same waveform.

In various implementations, the flow monitor 1700 includes at least one transceiver 1716 configured to communicate with at least one external device 1718 over one or more communication networks 1720. Any communication network described herein can be included in the communication network(s) 1720 illustrated in FIG. 17. The external device(s) 1718, for example, includes at least one of a mechanical chest compression device, a monitor-defibrillator, an AED, an ECMO device, a ventilation device, a subject monitor, a mobile phone, a server, or a computing device. In some implementations, the transceiver(s) 1716 is configured to communicate with the external device(s) 1718 by transmitting and/or receiving signals in a wired fashion and/or wirelessly. For example, the transceiver(s) 1716 includes a NIC, a network adapter, a LAN adapter, or a physical, virtual, or logical address to connect to the various external devices and/or systems. In various examples, the transceiver(s) 1716 includes any sort of wireless transceivers capable of engaging in wireless communication (e.g., RF communication). For example, the communication network(s) 1720 includes one or more wireless networks that include a 3GPP network, such as an LTE RAN (e.g., over one or more LTE bands), an NR RAN (e.g., over one or more NR bands), or a combination thereof. In some cases, the transceiver(s) 1716 includes other wireless modems, such as a modem for engaging in WI-FI^{®}, WIGIG^{®}, WIMAX^{®}, BLUETOOTH^{®}, or infrared communication over the communication network(s) 1720. The signals, in various cases, encode data in the form of data packets, datagrams, or the like. In some cases, the signals are transmitted as compressions are being administered by the flow monitor 1700 (e.g., for real-time feedback by the external device(s) 1718), after compressions are administered by the flow monitor 1700 (e.g., for post-event review at the external device 1718), or a combination thereof.

In various cases, the processor(s) 1714 generates the control signal based on data encoded in the signals received from the external device(s) 1718. For instance, the signals include an instruction to initiate monitoring, and the processor(s) 1714 outputs a control signal that causes the transmitter(s) 1702 to emit the incident beam.

In some cases, the flow monitor 1700 includes at least one input device 1722. In various examples, the input device(s) 1722 is configured to receive an input signal from a user 1724, who may be a rescuer treating the subject 1708. Examples of the input device(s) 1722 include, for instance, a a keypad, a cursor control, a touch-sensitive display, a voice input device (e.g., a microphone), a haptic feedback device (e.g., a gyroscope), or any combination thereof. In various implementations, the processor(s) 1714 generate the control signal based on the input signal. For instance, the processor(s) 1714 generate the control signal to adjust a frequency of the compressions based on the flow monitor 1700 detecting a selection by the user 1724 of a user interface element displayed on a touchscreen or detecting the user 1724 pressing a button integrated with an external housing of the flow monitor 1700.

According to some examples, the input device(s) 1722 include one or more sensors. The sensor(s), for example, is configured to detect a physiological parameter of the subject 1708. In some cases, the sensor(s) include one or more microphones, one or more accelerometers, one or more oximetry sensors, or one or more ECG sensors. The sensor(s), for instance, is configured to detect one or more physiological parameters. In some implementations, the sensor(s) is configured to detect a state parameter of the flow monitor 1700, such as a position of the flow monitor 1700 with respect to the subject 1708 or the like. According to some implementations, the signals transmitted by the transceiver(s) 1716 indicate the physiological parameter(s) and/or the state parameter(s).

The flow monitor 1700 further includes at least one output device 1725, in various implementations. Examples of the output device(s) 1725 include, for instance, least one of a display (e.g., a projector, an LED screen, etc.), a speaker, a haptic output device, a printer, a light such as an LED, or any combination thereof. In some implementations, the output device(s) 1725 include a screen configured to display various parameters detected by and/or reported to the flow monitor 1700, a battery level of the flow monitor 1700, and other relevant information.

The flow monitor 1700 further includes memory 1726. In various implementations, the memory 1726 is volatile (such as RAM), non-volatile (such as ROM, flash memory, etc.) or some combination of the two. The memory 1726 stores instructions that, when executed by the processor(s) 1714, causes the processor(s) 1714 to perform various operations. In various examples, the memory 1726 stores methods, threads, processes, applications, objects, modules, any other sort of executable instruction, or a combination thereof. In some cases, the memory 1726 stores files, databases, or a combination thereof. In some examples, the memory 1726 includes, but is not limited to, RAM, ROM, 16PROM, flash memory, or any other memory technology. In some examples, the memory 1726 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 1726 stores instructions, programs, threads, objects, data, or any combination thereof, that cause the processor(s) 1714 to perform various functions. In various cases, the memory 1726 stores one or more parameters that are detected by the flow monitor 1700 and/or reported to the flow monitor 1700.

In implementations of the present disclosure, the memory 1726 also stores one or more components 1728. The component(s) 1728 include programs, instructions, files, databases, models, or any other type of data that causes the flow monitor 1700 to perform any of the functions described herein.

### EXAMPLE CLAUSES

1. A flow detector, including: a transducer including: a piezoelectric crystal with a cross-section including a curved surface, the piezoelectric crystal being configured to emit ultrasound from the curved surface toward a blood vessel in a first direction; and a receiver configured to detect a reflection of the ultrasound from blood flowing in a blood vessel in a second direction, the first direction being non-perpendicular to the second direction; a processor configured to determine a velocity of the blood by analyzing the reflection; and a display configured to visually output an indication of the velocity of the blood.
2. The flow detector of clause 1, further including: a housing configured to enclose the piezoelectric crystal, the piezoelectric crystal, the receiver, and the processor, the housing including a surface configured to contact skin, wherein the first direction is non-perpendicular to the surface of the housing, and wherein the piezoelectric crystal is configured to emit the ultrasound through the surface of the housing.
3. The flow detector of clause 1 or 2, wherein the receiver includes the piezoelectric crystal.
4. A transducer, including: a non-cubic piezoelectric crystal including an emitting side configured to emit ultrasound toward a blood vessel; and a receiver configured to detect a reflection of the ultrasound from blood in a blood vessel.
5. The transducer of clause 4, wherein the non-cubic piezoelectric crystal includes zirconate titanate, barium titanate, or lithium niobate.
6. The transducer of clause 4 or 5, wherein the non-cubic piezoelectric crystal has a polygonal cross-section, the polygonal cross-section including greater than four sides, and wherein the emitting side of the non-cubic piezoelectric crystal includes one of the greater than four sides of the polygonal cross-section.
7. The transducer of any of clauses 4 to 6, wherein the non-cubic piezoelectric crystal includes a circular cross-section, and wherein the emitting side of the non-cubic piezoelectric crystal includes a curved side of the circular cross-section.
8. The transducer of any of clauses 4 to 7, wherein the non-cubic piezoelectric crystal includes a cross-section with a curved side, and wherein the emitting side of the non-cubic piezoelectric crystal includes the curved side of the cross-section.
9. The transducer of any of clauses 4 to 8, wherein the non-cubic piezoelectric crystal is configured to emit the ultrasound in a first direction, and wherein the blood in the blood vessel is flowing in a second direction that is non-perpendicular to the first direction.
10. The transducer of clause 9, further including: a housing configured to enclose the non-cubic piezoelectric crystal and the receiver, the housing including a surface configured to contact skin, wherein the first direction is non-perpendicular to the surface of the housing, and wherein the non-cubic piezoelectric crystal is configured to emit the ultrasound through the surface of the housing.
11. The transducer of any of clauses 4 to 10, further including: an analog-to-digital converter (ADC) configured to generate a digital signal indicative of the reflection.
12. The transducer of any of clauses 4 to 11, further including: a processor configured to determine a velocity of the blood by determining a difference between a frequency of the ultrasound emitted by the non-cubic piezoelectric crystal and a frequency of the reflection of the ultrasound from the blood in the blood vessel.
13. A method, including: emitting, from a side of a non-cubic piezoelectric crystal, ultrasound toward a blood vessel and in a first direction; receiving a reflection of the ultrasound from blood flowing through the blood vessel in a second direction, the first direction being non-perpendicular to the second direction; determining a velocity of the blood by analyzing the reflection of the ultrasound from the blood flowing through the blood vessel; and outputting an indication of the velocity.
14. The method of clause 13, wherein the non-cubic piezoelectric crystal has a polygonal cross-section, the polygonal cross-section including greater than four sides, and wherein the side of the non-cubic piezoelectric crystal includes one of the greater than four sides.
15. The method of clause 13 or 14, wherein the non-cubic piezoelectric crystal includes a circular cross-section, and wherein the side of the non-cubic piezoelectric crystal includes a curved side of the circular cross-section.
16. The method of any of clauses 13 to 15, wherein the non-cubic piezoelectric crystal includes a cross-section with a curved side, and wherein the side of the non-cubic piezoelectric crystal includes the curved side of the cross-section.
17. The method of any of clauses 13 to 16, wherein emitting, from the side of the non-cubic piezoelectric crystal, ultrasound toward the blood vessel and in the first direction includes: transmitting the ultrasound through a surface of a housing disposed on skin, the surface of the housing being substantially parallel to the second direction.
18. The method of any of clauses 13 to 17, wherein determining the velocity of the blood by analyzing the reflection of the ultrasound from the blood flowing through the blood vessel includes: determining a difference between a characteristic of the ultrasound and a characteristic of the reflection of the ultrasound, the characteristic being a frequency or rate of change of phase.
19. The method of any of clauses 13 to 18, wherein outputting the indication of the velocity includes: determining that the velocity is above a first threshold or is below a second threshold; and in response to determining that the velocity is above the first threshold or is below the second threshold, outputting an alert.
20. The method of any of clauses 13 to 19, wherein outputting the indication of the velocity includes: outputting an audible signal indicating the velocity; outputting a visual signal indicating the velocity; and/or outputting, to an external device, a communication signal indicating the velocity.
21. A method, including: determining a velocity of blood flowing through a vessel by analyzing information representative of ultrasound emitted toward a blood vessel and information representative of a reflection of the ultrasound from the blood flowing through the blood vessel; and outputting an indication of the velocity.
22. The method of clause 21, wherein the information representative of the reflection of the ultrasound is received from a flow detector according to any of clauses 1 to 3 or a transducer according to any of clauses 4 to 12.
23. The method of clause 21 or 22, wherein determining the velocity of the blood by analyzing the information representative of the reflection of the ultrasound from the blood flowing through the blood vessel includes: determining a difference between a characteristic of the ultrasound and a characteristic of the reflection of the ultrasound, the characteristic being a frequency or rate of change of phase.
24. The method of any of clauses 21 to 23, wherein outputting the indication of the velocity includes: determining that the velocity is above a first threshold or is below a second threshold; and in response to determining that the velocity is above the first threshold or is below the second threshold, outputting an alert.
25. The method of any of clauses 21 to 24, wherein outputting the indication of the velocity includes: outputting an audible signal indicating the velocity; outputting a visual signal indicating the velocity; and/or outputting, to an external device, a communication signal indicating the velocity.26. A system, including: a flow detector including: a housing including a surface configured to contact skin of a subject; an emitter including a planar face that is non-parallel to the surface of the housing, the emitter being configured to emit, from the planar face, an ultrasound signal through the surface of the housing, toward a blood vessel of the subject, and in a direction that is non-perpendicular to the surface of the housing; a wedge disposed between the surface of the housing and the planar face of a transducer, the wedge including an acoustically transparent material; a receiver configured to detect a reflection of the ultrasound signal from blood in the blood vessel; and a processor configured to determine a velocity of the blood by analyzing data indicative of the reflection of the ultrasound signal from the blood in the blood vessel; and a monitor communicatively coupled to the flow detector and configured to output a signal indicating the velocity of the blood.
27. The system of clause 26, wherein the monitor is further configured to: determine that the velocity of the blood is greater than a threshold; and in response to determining that the velocity of the blood is greater than the threshold, output a signal indicating that the blood is spontaneously circulating in the subject.
28. The system of clause 26 or 27, wherein the emitter includes a first piezoelectric crystal, and wherein the receiver includes the first piezoelectric crystal or a second piezoelectric crystal.
29. A flow detector, including: a housing including a surface configured to contact skin of a subject; an emitter configured to emit an ultrasound signal through the surface of the housing, toward a blood vessel of the subject, and in a direction that is non-perpendicular to the surface of the housing; a receiver configured to detect a reflection of the ultrasound signal from blood in the blood vessel; and a processor configured to determine a velocity of the blood by analyzing data indicative of the reflection of the ultrasound signal from the blood in the blood vessel.
30. The flow detector of clause 29, wherein the emitter includes: a piezoelectric crystal configured to emit the ultrasound signal from a face of the piezoelectric crystal, the face of the piezoelectric crystal being non-parallel to the surface of the housing.
31. The flow detector of clause 30, further including: a spacer disposed between the face of the piezoelectric crystal and the surface of the housing.
32. The flow detector of clause 31, wherein the spacer includes silicone, polyvinyl alcohol (PVA), or polyacrylamide (PAA).
33. The flow detector of any of clauses 29 to 32, further including: a piezoelectric crystal configured to emit the ultrasound signal; and a spacer disposed between the piezoelectric crystal and the surface of the housing, the spacer including materials that conduct the ultrasound signal at different speeds.
34. The flow detector of clause 33, wherein the materials include polyethylene (PE) and polymethyl-methacrylate (PMMA), or wherein the materials include polyurethane and RTV.
35. The flow detector of any of clauses 29 to 34, the ultrasound signal being first ultrasound, wherein the emitter includes: a first piezoelectric crystal configured to emit the first ultrasound; and a second piezoelectric crystal configured to emit the second ultrasound, wherein the receiver is further configured to detect a reflection of the second ultrasound, and wherein the processor is further configured to: select the first ultrasound signal by comparing the reflection of the first ultrasound signal and the reflection of the second ultrasound signal; and in response to selecting the first ultrasound, causing the emitter to deactivate the second piezoelectric crystal.
36. The flow detector of any of clauses 29 to 35, wherein the processor is configured to determine the velocity of the blood by determining a difference between a characteristic of the ultrasound signal and a characteristic of the reflection of the ultrasound signal from the blood in the blood vessel, the characteristic including a frequency or rate of change of phase.
37. The flow detector of any of clauses 29 to 36, further including: an output device configured to output an indication of the velocity of the blood.
38. A method, including: emitting ultrasound signal through a portion of skin of a subject and toward a blood vessel of the subject, the ultrasound signal being emitted in a direction that is non-perpendicular to the portion of skin of the subject; detecting a reflection of the ultrasound signal from blood in the blood vessel; determining a velocity of the blood by analyzing the reflection of the ultrasound signal from the blood in the blood vessel; and outputting an indication of the velocity of the blood.
39. The method of clause 38, wherein emitting the ultrasound signal through the portion of the skin of the subject and toward the blood vessel of the subject includes emitting the ultrasound signal from a face of a piezoelectric crystal, the face of the piezoelectric crystal being non-parallel to the portion of the skin of the subject.
40. The method of clause 38 or 39, wherein emitting the ultrasound signal through the portion of the skin of the subject and toward the blood vessel of the subject includes: transmitting, from a face of a piezoelectric crystal, the ultrasound signal through a spacer disposed between the face of the piezoelectric crystal and the portion of the skin of the subject.
41. The method of clause 40, wherein the spacer includes polyethylene (PE), polymethyl-methacrylate (PMMA), silicone, polyvinyl alcohol (PVA), polyvinyl chloride (PVC), or polyacrylamide (PAA).
42. The method of clause 40 or 41, wherein transmitting the ultrasound signal through the spacer disposed between the face of the piezoelectric crystal and the portion of the skin of the subject includes bending, by the spacer, the ultrasound, and wherein the spacer includes materials that conduct the ultrasound signal at different speeds.
43. The method of clause 42, wherein the materials include polyethylene (PE) and polymethyl-methacrylate (PMMA), or wherein the materials include polyurethane and RTV.
44. The method of any of clauses 38 to 43, wherein determining the velocity of the blood by analyzing the reflection of the ultrasound signal from the blood in the blood vessel includes determining a difference between a characteristic of the ultrasound signal and a characteristic of the reflection of the ultrasound signal from the blood in the blood vessel, the characteristic including a frequency or rate of change of phase.
45. The method of any of clauses 38 to 44, further including: determining that the velocity of the blood is greater than a first threshold or lower than a second threshold; and in response to determining that the velocity of the blood is greater than the first threshold or lower than the second threshold, generating an alert, wherein the indication of the velocity includes the alert.
46. A method, including: determining a velocity of blood flowing through a vessel by analyzing information representative of ultrasound emitted toward a blood vessel and information representative of a reflection of the ultrasound from the blood flowing through the blood vessel; and outputting an indication of the velocity.
47. The method of clause 46, wherein the information representative of the reflection of the ultrasound is received from a flow detector according to any of clauses 1 to 3, a transducer according to any of clauses 4 to 12, a system according to any of clauses 26-28, or a flow detector according to any of clauses 29-37.
48. The method of clause 46 or 47, wherein determining the velocity of the blood by analyzing the information representative of the reflection of the ultrasound signal from the blood in the blood vessel includes determining a difference between a characteristic of the ultrasound signal and a characteristic of the reflection of the ultrasound signal from the blood in the blood vessel, the characteristic including a frequency or rate of change of phase.
45. The method of any of clauses 38 to 44, further including: determining that the velocity of the blood is greater than a first threshold or lower than a second threshold; and in response to determining that the velocity of the blood is greater than the first threshold or lower than the second threshold, generating an alert, wherein the indication of the velocity includes the alert.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

The subject matter of the claims of the parent application is repeated below as numbered embodiments E1 to E15, which are not claims, in order to fully retain basis for this subject matter in the present application.
E1. A transducer, comprising:
   a non-cubic piezoelectric crystal comprising an emitting side configured to emit ultrasound toward a blood vessel; and
   a receiver configured to detect a reflection of the ultrasound from blood in a blood vessel.
E2. The transducer of E1, wherein the non-cubic piezoelectric crystal comprises zirconate titanate, barium titanate, or lithium niobate.
E3. The transducer of E1 or E2, wherein the non-cubic piezoelectric crystal has a polygonal cross-section, the polygonal cross-section comprising greater than four sides, and
   wherein the emitting side of the non-cubic piezoelectric crystal comprises one of the greater than four sides of the polygonal cross-section;or wherein the non-cubic piezoelectric crystal comprises a circular cross-section.
E4. The transducer of E1, E2 or E3, wherein the non-cubic piezoelectric crystal comprises a cross-section with a curved side, and
   wherein the emitting side of the non-cubic piezoelectric crystal comprises the curved side of the cross-section.
E5. The transducer of any of E1-E4, wherein the non-cubic piezoelectric crystal is configured to emit the ultrasound in a first direction, and
   wherein the blood in the blood vessel is flowing in a second direction that is non-perpendicular to the first direction.
E6. The transducer of E5, further comprising:
   a housing configured to enclose the non-cubic piezoelectric crystal and the receiver, the housing comprising a surface configured to contact skin,
   wherein the first direction is non-perpendicular to the surface of the housing, and
   wherein the non-cubic piezoelectric crystal is configured to emit the ultrasound through the surface of the housing.
E7. The transducer of any of E1-E6, further comprising:
   an analog-to-digital converter (ADC) configured to generate a digital signal indicative of the reflection.
E8. The transducer of any of E1-E7, further comprising:
   a processor configured to determine a velocity of the blood by determining a difference between a frequency of the ultrasound emitted by the non-cubic piezoelectric crystal and a frequency of the reflection of the ultrasound from the blood in the blood vessel.
E9. A flow detector, comprising the transducer of any of E1-E8.
E10. A method, comprising:
   emitting, from a side of a non-cubic piezoelectric crystal, ultrasound toward a blood vessel and in a first direction;
   receiving a reflection of the ultrasound from blood flowing through the blood vessel in a second direction, the first direction being non-perpendicular to the second direction;
   determining a velocity of the blood by analyzing the reflection of the ultrasound from the blood flowing through the blood vessel; and
   outputting an indication of the velocity.
E11. The method of E10, wherein the non-cubic piezoelectric crystal has a polygonal cross-section, the polygonal cross-section comprising greater than four sides, and
   wherein the side of the non-cubic piezoelectric crystal comprises one of the greater than four sides; or wherein the non-cubic piezoelectric crystal comprises a circular cross-section.
E12. The method of E10 or E11, wherein the non-cubic piezoelectric crystal comprises a cross-section with a curved side, and
   wherein the side of the non-cubic piezoelectric crystal comprises the curved side of the cross-section.
E13. The method of claim E10, E11 or E12, wherein emitting, from the side of the non-cubic piezoelectric crystal, ultrasound toward the blood vessel and in the first direction comprises:
   transmitting the ultrasound through a surface of a housing disposed on skin, the surface of the housing being substantially parallel to the second direction.
E14. The method of any of E10-E13, wherein determining the velocity of the blood by analyzing the reflection of the ultrasound from the blood flowing through the blood vessel comprises:
   determining a difference between a characteristic of the ultrasound and a characteristic of the reflection of the ultrasound, the characteristic being a frequency or rate of change of phase.
E15. The method of any of E10-E14, wherein outputting the indication of the velocity comprises:
   determining that the velocity is above a first threshold or is below a second threshold; and
   in response to determining that the velocity is above the first threshold or is below the second threshold, outputting an alert; and/or wherein outputting the indication of the velocity comprises:
      outputting an audible signal indicating the velocity;
      outputting a visual signal indicating the velocity; and/or
      outputting, to an external device, a communication signal indicating the velocity.

## Claims

1. A transducer, comprising:
a housing comprising a surface configured to contact skin of a subject;
an emitter configured to emit an ultrasound signal through the surface of the housing, toward a blood vessel of the subject, and in a direction that is non-perpendicular to the surface of the housing;
a receiver configured to detect a reflection of the ultrasound signal from blood in the blood vessel; and
a spacer disposed between the emitter and the surface of the housing, the spacer comprising materials that conduct the ultrasound signal at different speeds.

2. The transducer of claim 1, wherein the spacer is wedge-shaped, and/or
wherein the spacer includes multiple layers that are in contact with the emitter.

3. The transducer of claim 2, wherein the emitter is tilted with respect to the skin of the subject.

4. The transducer of any one of claims 1-3, wherein the spacer is substantially acoustically transparent, and/or
wherein the spacer comprises a polymer or gel that is configured to perform impedance matching between the emitter and the skin.

5. The transducer of any one of claims 1-4, wherein the materials comprise polyethylene (PE), polymethyl-methacrylate (PMMA), silicone, polyvinyl alcohol (PVA), polyvinyl chloride (PVC), or polyacrylamide (PAA).

6. The transducer of any one of claims 1-5, further comprising:
an analog-to-digital converter (ADC) configured to generate a digital signal indicative of the reflection.

7. The transducer of any one of claims 1-6, further comprising:
a processor configured to determine a velocity of the blood by determining a difference between a frequency of the ultrasound signal emitted by the emitter and a frequency of the reflection of the ultrasound signal from the blood in the blood vessel.

8. A method, comprising:
transmitting, from an emitter, an ultrasound signal through a spacer disposed between the emitter and a portion of skin of a subject, the spacer comprising materials that conduct the ultrasound signal at different speeds;
outputting, by the spacer, the ultrasound signal toward a blood vessel of the subject in a direction that is non-perpendicular to the portion of skin of the subject;
detecting a reflection of the ultrasound signal from blood in the blood vessel;
determining a velocity of the blood by analyzing the reflection of the ultrasound signal from the blood in the blood vessel; and
outputting an indication of the velocity of the blood.

9. The method of claim 8, wherein the spacer is wedge-shaped, and/or
wherein the spacer includes multiple layers that are in contact with the emitter.

10. The method of claim 8 or 9, wherein the spacer is substantially acoustically transparent, and/or
wherein the spacer comprises a polymer or gel that is configured to perform impedance matching between the emitter and the skin of the subject.

11. The method of any one of claims 8-10, wherein the materials comprise PE, PMMA, silicone, PVA, PVC, or PAA.

12. The method of any one of claims 8-11, further comprising:
bending, by the spacer, the ultrasound signal.

13. The method of any one of claims 8-12, wherein outputting, by the spacer, the ultrasound signal toward the blood vessel of the subject comprises:
transmitting the ultrasound signal through a surface of a housing disposed on skin.

14. The method of any one of claims 8-13, wherein determining the velocity of the blood by analyzing the reflection of the ultrasound signal from the blood in the blood vessel comprises:
determining a difference between a characteristic of the ultrasound signal and a characteristic of the reflection of the ultrasound signal, the characteristic being a frequency of rate of change of phase.

15. The method of any one of claims 8-14, wherein outputting the indication of the velocity comprises:
determining that the velocity is above a first threshold or below a second threshold; and
in response to determining that the velocity is above the first threshold or below the second threshold, outputting an alert, and/or
wherein outputting the indication of the velocity comprises:
outputting an audible signal indicating the velocity;
outputting a visual signal indicating the velocity; or
outputting, to an external device, a communication signal indicating the velocity.
